# EUROPEAN PATENT APPLICATION

(11) **EP 4 768 019 A1**
(43) Date of publication of application: **01.07.2026**
(21) Application number: 25227466.7
(22) Date of filing: 29.12.2025
(51) Int. Cl.: A61K 9/08, A61K 47/22, A61K 47/38, A61K 47/40, A61P 27/02

(54) **EYE DROP PLATFORM FOR DELIVERING ACTIVE INGREDIENTS TO ANTERIOR CHAMBER TISSUE AND/OR POSTERIOR CHAMBER TISSUE OF EYE**

(30) Priority: 30.12.2024 US 202463739976 P
(71) Applicant: Industrial Technology Research Institute, 310401 Hsinchu (TW)
(72) Inventor: Cheng, Felice, Hsinchu County (TW); Lo, Yu-Wen, New Taipei City (TW); Chen, Chia-Ching, Taichung City (TW); Yin, Shao-Chan, Hsinchu City (TW); Wang, Wen-Yu, Taoyuan City (TW); Lin, Yu-Chin, Taichung City (TW); Chang, Li-Wen, Taoyuan City (TW); Kuo, Po-Hsien, Hsinchu County (TW); Yeh, Nien-Tzu, Tainan City (TW); Cheng, Ping-Fu, Hsinchu County (TW); Chen, Sung-En, Hsinchu County (TW); Ma, Ling-Jen, Hsinchu City (TW); Hsieh, Tai-Ju, Taichung City (TW)
(74) Representative: Fuchs Patentanwälte Partnerschaft mbB

(57) **Abstract**

An eye drop platform for delivering active ingredients to anterior chamber tissue and/or posterior chamber tissue of eye is provided. The eye drop platform for delivering active ingredients to anterior chamber tissue and/or posterior chamber tissue of eye includes a delivery composition and a pharmaceutically acceptable solvent for dissolving the delivery composition to form a solution as the eye drop platform for delivering active ingredients to anterior chamber tissue and/or posterior chamber tissue of eye. The delivery composition includes cyclodextrin and/or a derivative thereof, and caffeine. The concentration of the cyclodextrin and/or the derivative thereof in the solution ranges from 5-500 mg/mL, and the concentration of the caffeine in the solution ranges from 0.5-50 mg/mL. The active ingredient is a hydrophilic compound, and the hydrophilic compound has a water solubility greater than 1 mg/mL.

## Description

### TECHNICAL FIELD

The present disclosure relates to an eye drop platform for delivering an active ingredient, and, in particular, it relates to an eye drop platform for delivering an active ingredient to anterior chamber tissue and/or posterior chamber tissue of an eye, and uses thereof.

### BACKGROUND

Structure of an eye can be divided into an anterior chamber (including, for example, a tear film, a cornea, a pupil, a lens, and a ciliary body) and a posterior chamber (including, for example, a sclera, a choroid, a retina, a vitreous humor, and an optic nerve).

The eye has multiple natural barriers that limit the ability of drugs to penetrate and reach target lesion sites. In particular, even for drugs having high water solubility, only a very limited amount can be delivered to lesion sites in the posterior chamber of the eye.

As a result, treatment of posterior chamber diseases often relies on intraocular injection of drugs. However, invasive treatments involve a high risk of infection, and injection-related fear and need for regular clinical visits result in poor patient compliance. Conventional eye drops, on the other hand, have limited applicability as drugs are difficult to deliver to posterior chamber tissue.

Accordingly, there remains a need to develop a non-invasive drug delivery platform that can effectively deliver drugs to anterior chamber tissue and/or posterior chamber tissue of the eye.

### SUMMARY

The present disclosure provides an eye drop platform for delivering an active ingredient to anterior chamber tissue and/or posterior chamber tissue of an eye, comprising: a delivery composition and a pharmaceutically acceptable solvent for dissolving the delivery composition to form a solution. The solution serves as the eye drop platform for delivering the active ingredient to anterior chamber tissue and/or posterior chamber tissue of the eye. The delivery composition includes cyclodextrin and/or a derivative thereof and caffeine. A concentration of the cyclodextrin and/or the derivative thereof in the solution ranges from 5-500 mg/mL; and a concentration of the caffeine in the solution ranges from 0.5-50 mg/mL. The active ingredient is a hydrophilic compound having a water solubility greater than 1 mg/mL.

Further, the present disclosure provides an eye drop for improving exposure of an active ingredient in anterior chamber tissue and/or posterior chamber tissue of an eye, comprising: a delivery composition and a pharmaceutically acceptable solvent for dissolving the delivery composition to form a solution. The solution serves as the eye drop for improving exposure of an active ingredient in anterior chamber tissue and/or posterior chamber tissue of an eye. The delivery composition comprises cyclodextrin and/or a derivative thereof; and caffeine. A concentration of the cyclodextrin and/or the derivative thereof in the eye drop ranges from 5-500 mg/mL; and a concentration of the caffeine in the eye drop ranges from 0.5-50 mg/mL. The active ingredient is a hydrophilic compound, and the hydrophilic compound has a water solubility greater than 1 mg/mL.

In addition, the present disclosure provides a composite formulation comprising: an active ingredient, a delivery composition, and a pharmaceutically acceptable solvent for dissolving the active ingredient and the delivery composition to form a solution. The solution serves as the composite formulation. The delivery composition includes cyclodextrin and/or a derivative thereof; and caffeine. The active ingredient is a hydrophilic compound, and the hydrophilic compound has a water solubility greater than 1 mg/mL. A concentration of the active ingredient in the solution ranges from 0.05-500 mg/mL, a concentration of the cyclodextrin and/or the derivative thereof in the solution ranges from 5-500 mg/mL, and a concentration of the caffeine in the solution ranges from 0.5-50 mg/mL. Further, the composite formulation is an eye drop.

The present disclosure also provides an eye drop for treating and/or preventing an anterior chamber disease and/or a posterior chamber disease of an eye, comprising: the composite formulation described above.

Further, the present disclosure further provides an eye drop for treating and/or preventing age-related macular degeneration, comprising: a composite formulation which comprises sodium phenylbutyrate; a delivery composition, and a pharmaceutically acceptable solvent for dissolving the sodium phenylbutyrate and the delivery composition to form a solution, and the solution serves as the composite formulation. The delivery composition includes (2-hydroxypropyl)-β-cyclodextrin and/or (2-hydroxypropyl)-γ-cyclodextrin; and caffeine. A concentration of the sodium phenylbutyrate in the solution ranges from 0.05-150 mg/mL, a concentration of the (2-hydroxypropyl)-β-cyclodextrin and/or the (2-hydroxypropyl)-γ-cyclodextrin in the solution ranges from 5-500 mg/mL, and a concentration of the caffeine in the solution ranges from 0.5-50 mg/mL.

### BRIEF DESCRIPTION OF THE DRAWINGS

The present invention can be more fully understood by reading the subsequent detailed description and examples with references made to the accompanying drawings, wherein:

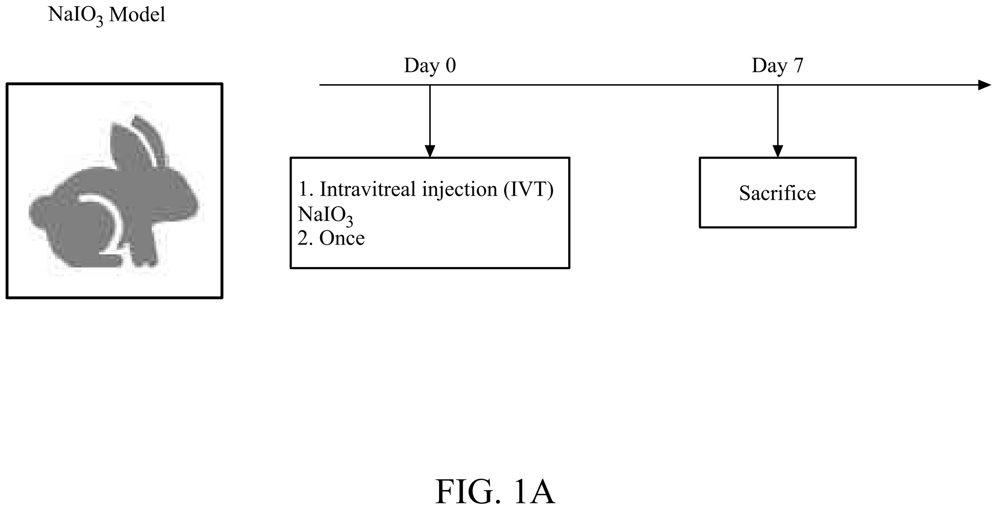

FIG. 1A illustrates a procedure of a NaIO₃-induced animal disease model experiment according to one embodiment of the present disclosure;

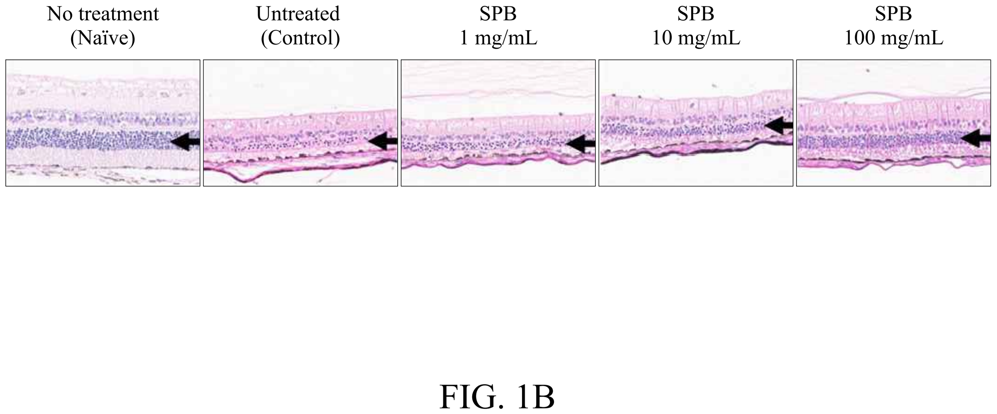

FIG. 1B illustrates hematoxylin and eosin (H&E) staining results of the outer nuclear layer (ONL) of rabbit retinas in the NaIO₃-induced animal disease model experiment according to one embodiment of the present disclosure;

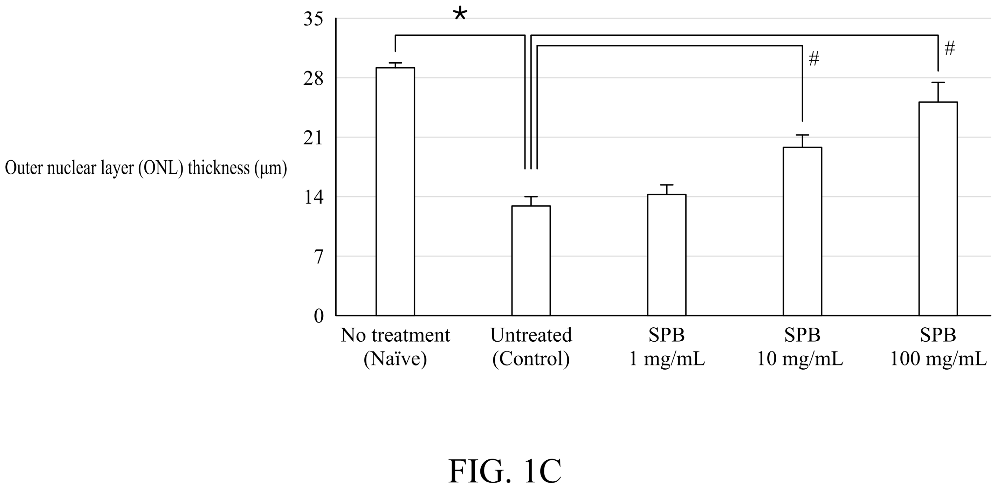

FIG. 1C illustrates the thickness of the outer nuclear layer of rabbit retinas in the NaIO₃-induced animal disease model experiment according to one embodiment of the present disclosure. N ≥ 6, and values are presented as mean ± standard error of the mean (SEM); *: untreated group compared with the non-treated control group, p < 0.05; #: sodium phenylbutyrate (SPB)-treated group compared with the untreated group, p < 0.05;

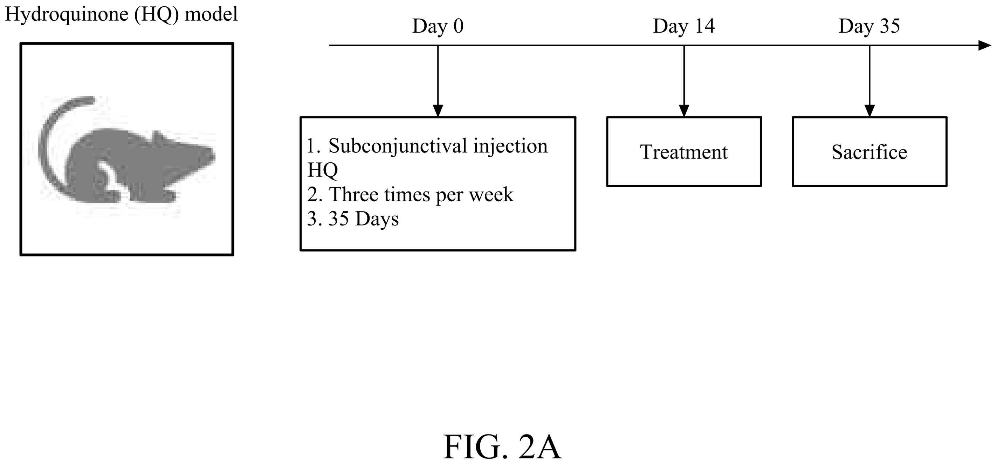

FIG. 2A illustrates a procedure of a hydroquinone (HQ)-induced animal disease model experiment according to one embodiment of the present disclosure;

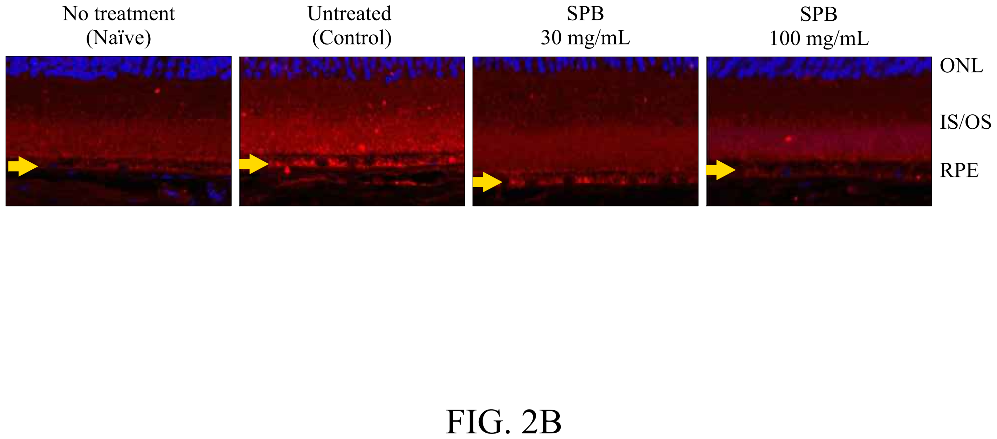

FIG. 2B illustrates immunofluorescence staining results of mouse eye samples in the hydroquinone (HQ)-induced animal disease model experiment according to one embodiment of the present disclosure. Arrows indicate expression of clusterin. ONL: outer nuclear layer; IS/OS: photoreceptor inner segment/outer segment junction; RPE: retinal pigment epithelium;

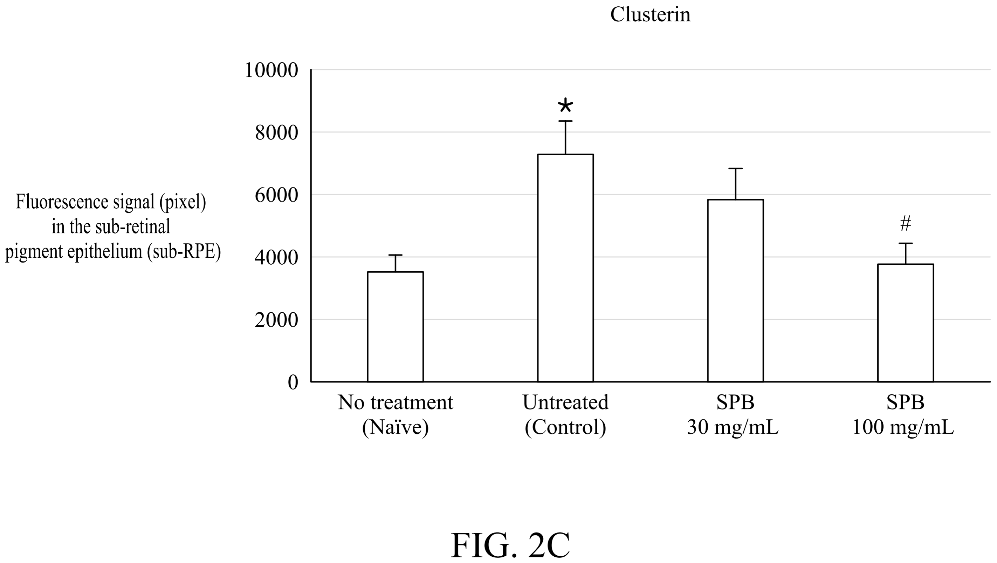

FIG. 2C illustrates expression level of clusterin in mouse eye samples in a hydroquinone (HQ)-induced animal disease model experiment according to one embodiment of the present disclosure. N ≥ 6, and values are presented as mean ± standard error of the mean (SEM). *: control group (untreated group) compared with the non-treated group, p *<* 0.05; #: sodium phenylbutyrate (SPB)-treated group compared with the control group (untreated group), p < 0.05;

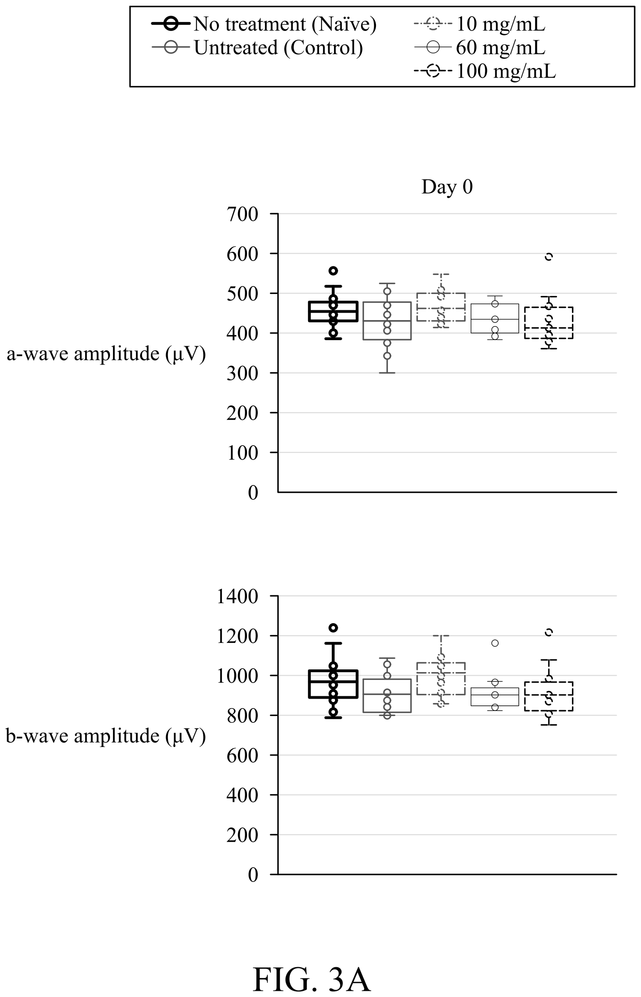

FIG. 3A illustrates electroretinogram (ERG) signals (a-wave/b-wave) of mice on Day 0 in a hydroquinone (HQ)-induced animal disease model experiment according to one embodiment of the present disclosure. N ≥ 14, and values are presented as mean ± standard error of the mean (SEM). a-wave: photoreceptors; b-wave: inner nuclear layer (INL);

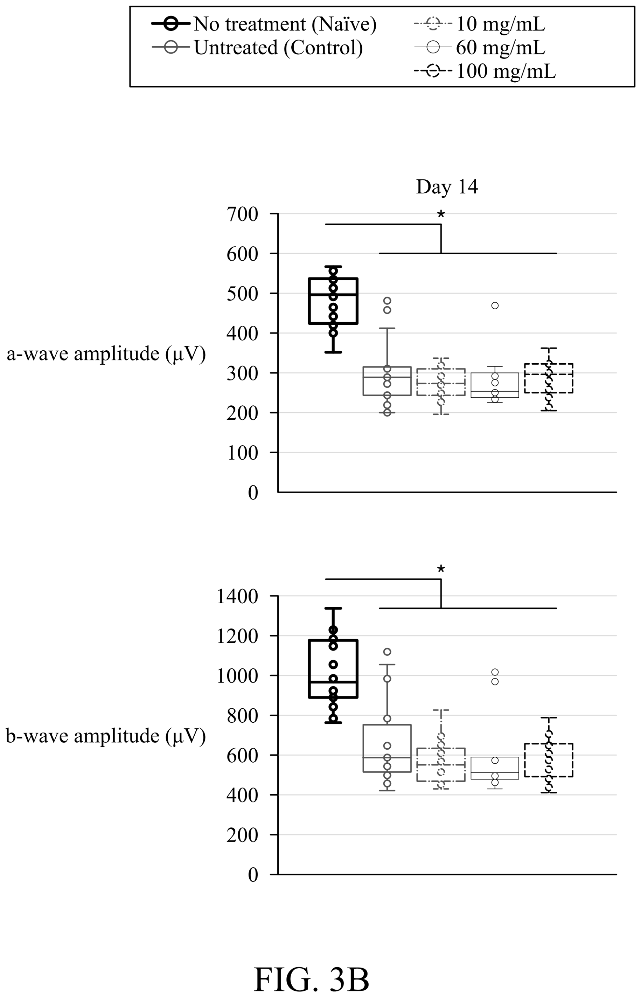

FIG. 3B illustrates electroretinogram (ERG) signals (a-wave/b-wave) of mice on Day 14 in a hydroquinone (HQ)-induced animal disease model experiment according to one embodiment of the present disclosure. N ≥ 14, and values are presented as mean ± standard error of the mean (SEM). a-wave: photoreceptors; b-wave: inner nuclear layer (INL). *: *p <* 0.05, indicating a statistically significant difference; and

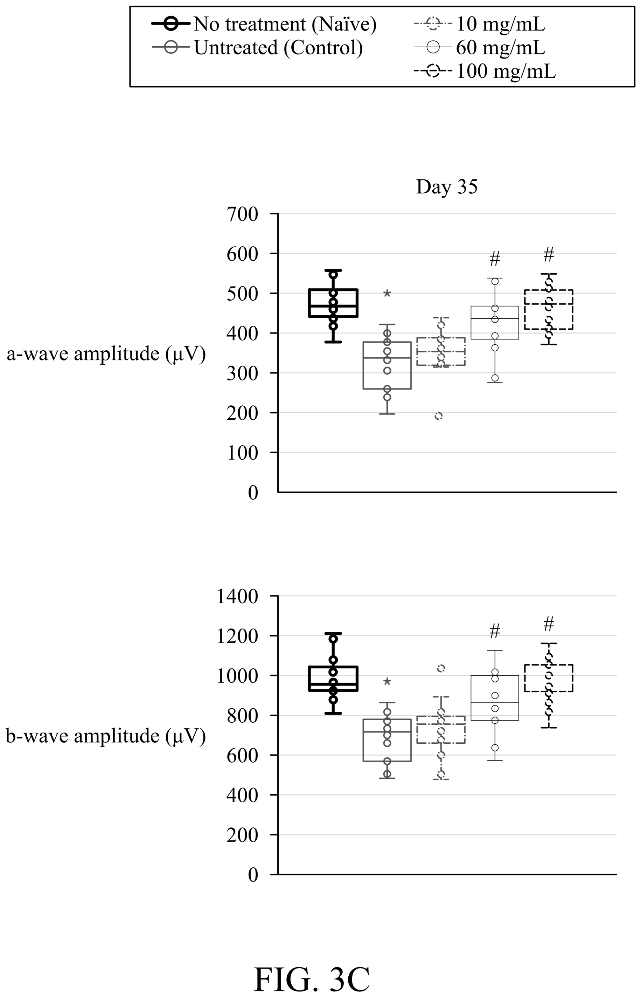

FIG. 3C illustrates electroretinogram (ERG) signals (a-wave/b-wave) of mice on Day 35 in a hydroquinone (HQ)-induced animal disease model experiment according to one embodiment of the present disclosure. N ≥ 14, and values are presented as mean ± standard error of the mean (SEM); analysis of variance (ANOVA). *: untreated group compared with the non-treated group, *p* < 0.05; #: sodium phenylbutyrate (SPB)-treated group compared with the untreated group, p < 0.05. a-wave: photoreceptors; b-wave: inner nuclear layer (INL).

### DETAILED DESCRIPTION

The following description is made for the purpose of illustrating the general principles of the invention and should not be taken in a limiting sense. The scope of the invention is best determined by reference to the appended claims.

The present disclosure may provide an eye drop platform for delivering an active ingredient to anterior chamber tissue and/or posterior chamber tissue of an eye.

The active ingredient described herein may include, but is not limited to, a hydrophilic active ingredient. As used herein, the term "hydrophilic active ingredient" refers to any biologically active substance having a water solubility greater than about 1 mg/mL.

The hydrophilic active ingredient described above may include, but is not limited to, a drug having a neuroprotective function, a complement inhibitor, an AMP-activated protein kinase (AMPK) activator, a Rho-associated coiled-coil kinase inhibitor, or any combination thereof.

The drug having a neuroprotective function described above may include, but is not limited to, sodium phenylbutyrate (SPB), dexmedetomidine HCl, and tauro-ursodesoxy cholic acid (TUDCA), or any combination thereof.

The complement inhibitor described above may include, but is not limited to, iptacopan (LNP-023) and nafamostat mesylate (FUT-175), or any combination thereof.

Further, the AMP-activated protein kinase (AMPK) activator described above may include, but is not limited to, a biguanide drug. Examples of the biguanide drug described above may include, but are not limited to, metformin HCl.

Further, examples of the Rho-associated coiled-coil kinase inhibitor described above may include, but are not limited to, (S)-N-(4-(1H-pyrazol-4-yl)phenyl)-3-amino-2-phenylpropanamide dihydrochloride (ITRI-E-(S)4046). The (S)-N-(4-(1H-pyrazol-4-yl)phenyl)-3-amino-2-phenylpropanamide dihydrochloride described above is further a myosin light chain kinase 4 (MLCK4) inhibitor. In other words, (S)-N-(4-(1H-pyrazol-4-yl)phenyl)-3-amino-2-phenylpropanamide dihydrochloride is a dual-target drug targeting both Rho-associated coiled-coil kinase and myosin light chain kinase 4.

The eye drop platform for delivering the active ingredient to anterior chamber tissue and/or posterior chamber tissue of the eye described above is capable of effectively delivering hydrophilic drugs to posterior chamber tissue and increasing and/or improving exposure of the drugs in posterior chamber tissue when administered as eye drops, without requiring an invasive administration route, even for hydrophilic drugs that are otherwise difficult to deliver to the posterior chamber or exhibit low exposure therein.

The eye drop platform for delivering the active ingredient to anterior chamber tissue and/or posterior chamber tissue of the eye described above may include, but is not limited to, a delivery composition and a pharmaceutically acceptable solvent for dissolving the delivery composition to form a solution. The solution described above serves as the eye drop platform for delivering the active ingredient to anterior chamber tissue and/or posterior chamber tissue of the eye.

In the eye drop platform for delivering an active ingredient to anterior chamber tissue and/or posterior chamber tissue of the eye described above, the delivery composition may include, but is not limited to, cyclodextrin and/or a derivative thereof, and caffeine.

In one embodiment, a concentration of cyclodextrin and/or the derivative thereof in the solution described above may range from about 5 to 500 mg/mL, for example, about 10 to 475 mg/mL, about 15 to 450 mg/mL, about 20 to 425 mg/mL, about 25 to 400 mg/mL, about 5 mg/mL, about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 65 mg/mL, about 70 mg/mL, about 75 mg/mL, about 80 mg/mL, about 85 mg/mL, about 90 mg/mL, about 95 mg/mL, about 100 mg/mL, about 125 mg/mL, about 150 mg/mL, about 175 mg/mL, about 200 mg/mL, about 225 mg/mL, about 250 mg/mL, about 275 mg/mL, about 300 mg/mL, about 325 mg/mL, about 350 mg/mL, about 375 mg/mL, about 400 mg/mL, about 425 mg/mL, about 450 mg/mL, about 475 mg/mL, and about 500 mg/mL, but are not limited thereto.

The cyclodextrin described above may include, but is not limited to, α-cyclodextrin, β-cyclodextrin, γ-cyclodextrin, and δ-cyclodextrin, or any combination thereof.

The derivative of cyclodextrin described above may include, but is not limited to, hydroxypropyl-modified cyclodextrin, succinyl-modified cyclodextrin, and methyl-modified cyclodextrin, or any combination thereof. Examples of the hydroxypropyl-modified cyclodextrin may include, but are not limited to, (2-hydroxypropyl)-β-cyclodextrin and (2-hydroxypropyl)-γ-cyclodextrin, or any combination thereof.

Further, in one embodiment, a concentration of caffeine in the solution described above may range from about 0.5 to 50 mg/mL, for example, about 1 to 45 mg/mL, about 5 to 40 mg/mL, about 0.5 mg/mL, about 0.75 mg/mL, about 1 mg/mL, about 1.25 mg/mL, about 1.5 mg/mL, about 2 mg/mL, about 2.2 mg/mL, about 2.5 mg/mL, about 2.75 mg/mL, about 3 mg/mL, about 3.5 mg/mL, about 4 mg/mL, about 4.5 mg/mL, about 5 mg/mL, about 5.5 mg/mL, about 6 mg/mL, about 7 mg/mL, about 8 mg/mL, about 9 mg/mL, about 10 mg/mL, about 12 mg/mL, about 15 mg/mL, about 20 mg/mL, about 25 mg/mL, about 30 mg/mL, about 35 mg/mL, about 40 mg/mL, about 45 mg/mL, and about 50 mg/mL, but are not limited thereto.

Further, in one embodiment, a concentration of the delivery composition described above in the solution of the eye drop platform for delivering the active ingredient to anterior chamber tissue and/or posterior chamber tissue of the eye described above may range from about 6 to 570 mg/mL, for example, about 10 to 565 mg/mL, about 15 to 560 mg/mL, about 20 to 555 mg/mL, about 25 to 550 mg/mL, about 30 to 545 mg/mL, about 40 to 540 mg/mL, about 45 to 535 mg/mL, about 50 to 530 mg/mL, about 55 to 525 mg/mL, about 60 to 500 mg/mL, about 65 to 450 mg/mL, about 70 to 400 mg/mL, about 6 mg/mL, about 7 mg/mL, about 8 mg/mL, about 9 mg/mL, about 10 mg/mL, about 11 mg/mL, about 12 mg/mL, about 13 mg/mL, about 15 mg/mL, about 18 mg/mL, about 20 mg/mL, about 21 mg/mL, about 24 mg/mL, about 25 mg/mL, about 27 mg/mL, about 30 mg/mL, about 31 mg/mL, about 32 mg/mL, about 33 mg/mL, about 34 mg/mL, about 35 mg/mL, about 36 mg/mL, about 37 mg/mL, about 38 mg/mL, about 39 mg/mL, about 40 mg/mL, about 41 mg/mL, about 42 mg/mL, about 45 mg/mL, about 48 mg/mL, about 50 mg/mL, about 51 mg/mL, about 52 mg/mL, about 55 mg/mL, about 60 mg/mL, about 61 mg/mL, about 62 mg/mL, about 65 mg/mL, about 70 mg/mL, about 75 mg/mL, about 80 mg/mL, about 85 mg/mL, about 90 mg/mL, about 95 mg/mL, about 100 mg/mL, about 125 mg/mL, about 150 mg/mL, about 175 mg/mL, about 200 mg/mL, about 225 mg/mL, about 250 mg/mL, about 275 mg/mL, about 300 mg/mL, about 325 mg/mL, about 350 mg/mL, about 375 mg/mL, about 400 mg/mL, about 425 mg/mL, about 450 mg/mL, about 475 mg/mL, about 500 mg/mL, about 525 mg/mL, about 550 mg/mL, and about 570 mg/mL, but are not limited thereto.

Further, the pharmaceutically acceptable solvent described above in the eye drop platform for delivering an active ingredient to anterior chamber tissue and/or posterior chamber tissue of the eye described above may include, but is not limited to, water, saline, or any combination thereof.

In one embodiment, the delivery composition described above in the eye drop platform for delivering an active ingredient to anterior chamber tissue and/or posterior chamber tissue of the eye described above may further include, in addition to cyclodextrin and/or a derivative thereof and caffeine, a water-soluble polymer, but is not limited thereto. The addition of the water-soluble polymer can provide a more sustained delivery of the active ingredient to anterior chamber tissue and/or posterior chamber tissue of the eye.

In one embodiment, a concentration of the water-soluble polymer in the solution described above may range from about 0.5 to 20 mg/mL, for example, about 1 to 15 mg/mL, about 2 to 10 mg/mL, about 0.5 mg/mL, about 0.75 mg/mL, about 1 mg/mL, about 1.25 mg/mL, about 1.5 mg/mL, about 2 mg/mL, about 2.25 mg/mL, about 2.75 mg/mL, about 3 mg/mL, about 3.25 mg/mL, about 3.5 mg/mL, about 3.75 mg/mL, about 4 mg/mL, about 4.25 mg/mL, about 4.5 mg/mL, about 4.75 mg/mL, about 5 mg/mL, about 5.25 mg/mL, about 5.5 mg/mL, about 5.75 mg/mL, about 6 mg/mL, about 6.25 mg/mL, about 6.5 mg/mL, about 6.75 mg/mL, about 7 mg/mL, about 7.25 mg/mL, about 7.5 mg/mL, about 7.75 mg/mL, about 8 mg/mL, about 8.25 mg/mL, about 8.5 mg/mL, about 8.75 mg/mL, about 9 mg/mL, about 9.25 mg/mL, about 9.5 mg/mL, about 9.75 mg/mL, about 10 mg/mL, about 12.5 mg/mL, about 15 mg/mL, and about 20 mg/mL, but are not limited thereto.

The water-soluble polymer described above may include, but is not limited to, hydroxypropyl methyl cellulose (HPMC), hydroxypropyl cellulose, carboxymethyl cellulose (CMC), polyvinylpyrrolidone (PVP), polyvinyl alcohol, and poly(ethylene glycol)-poly(propylene glycol)-poly(ethylene glycol) (PEG-PPG-PEG) (ABA) triblock copolymers, or any combination thereof.

Based on the foregoing, the present disclosure further provides a use of a delivery composition in a preparation of an eye drop for delivering an active ingredient to anterior chamber tissue and/or posterior chamber tissue of an eye.

Similarly, based on the foregoing, the present disclosure further provides a method for delivering an active ingredient to anterior chamber tissue and/or posterior chamber tissue of an eye. The method may include, but is not limited to, administering an active ingredient to a subject in need thereof via an eye drop containing a delivery composition.

The use of the delivery composition in the preparation of the eye drop for delivering the active ingredient to anterior chamber tissue and/or posterior chamber tissue of the eye of the present disclosure, and the delivery composition in the method for delivering the active ingredient to anterior chamber tissue and/or posterior chamber tissue of the eye of the present disclosure described above, may be the same as the delivery composition in the eye drop platform for delivering the active ingredient to anterior chamber tissue and/or posterior chamber tissue of the eye of the present disclosure described above. Accordingly, all relevant descriptions of the delivery composition in the use and all relevant descriptions of the components thereof may be referred to the foregoing paragraphs related to the delivery composition and the components thereof in the eye drop platform for delivering the active ingredient to anterior chamber tissue and/or posterior chamber tissue of the eye of the present disclosure as described above, and are not repeated herein.

Further, descriptions relating to the use of the delivery composition in the preparation of the eye drop for delivering the active ingredient to anterior chamber tissue and/or posterior chamber tissue of the eye of the present disclosure and the active ingredient in the method for delivering the active ingredient to anterior chamber tissue and/or posterior chamber tissue of the eye of the present disclosure described above may be referred to all relevant descriptions regarding the active ingredient in the foregoing descriptions relating to the eye drop platform for delivering the active ingredient to anterior chamber tissue and/or posterior chamber tissue of the eye of the present disclosure, and are not repeated herein.

In addition, based on the foregoing, the present disclosure further provides a use of a delivery composition in the preparation of an eye drop for improving exposure of an active ingredient in anterior chamber tissue and/or posterior chamber tissue of an eye.

Similarly, based on the foregoing, the present disclosure may further provide an eye drop for improving exposure of an active ingredient in anterior chamber tissue and/or posterior chamber tissue of an eye. The eye drop may include, but is not limited to, a delivery composition and a pharmaceutically acceptable solvent for dissolving the delivery composition to form a solution. The solution serves as the eye drop for improving exposure of the active ingredient in anterior chamber tissue and/or posterior chamber tissue of the eye.

Likewise, based on the foregoing, the present disclosure may further provide a method for improving exposure of an active ingredient in anterior chamber tissue and/or posterior chamber tissue of an eye. The method may include, but is not limited to, administering an active ingredient to a subject in need thereof via an eye drop containing a delivery composition and a pharmaceutically acceptable solvent.

The use of the delivery composition in the preparation of the eye drop for improving exposure of the active ingredient in anterior chamber tissue and/or posterior chamber tissue of the eye of the present disclosure described above, the eye drop for improving exposure of the active ingredient in anterior chamber tissue and/or posterior chamber tissue of the eye of the present disclosure described above, and the delivery composition in the method for improving exposure of the active ingredient in anterior chamber tissue and/or posterior chamber tissue of the eye of the present disclosure described above may be the same as the delivery composition in the eye drop platform for delivering the active ingredient to anterior chamber tissue and/or posterior chamber tissue of the eye of the present disclosure described above. Accordingly, all relevant descriptions of the delivery composition and all relevant descriptions of the components thereof in the use, the eye drop, and the method may be referred to the foregoing paragraphs relating to the delivery composition and the components thereof in the eye drop platform for delivering the active ingredient to anterior chamber tissue and/or posterior chamber tissue of the eye of the present disclosure described above, and are not repeated herein.

Further, descriptions relating to the use of the delivery composition in the preparation of the eye drop for improving exposure of the active ingredient in anterior chamber tissue and/or posterior chamber tissue of the eye of the present disclosure, the eye drop for improving exposure of the active ingredient in anterior chamber tissue and/or posterior chamber tissue of the eye of the present disclosure, and the active ingredient in the method for improving exposure of the active ingredient in anterior chamber tissue and/or posterior chamber tissue of the eye of the present disclosure described above may be referred to all relevant descriptions of the active ingredient in the relevant descriptions of the eye drop platform of the present disclosure for delivering the active ingredient to anterior chamber tissue and/or posterior chamber tissue of the eye in the foregoing descriptions, and are not repeated herein.

The pharmaceutically acceptable solvent in the eye drop for improving exposure of the active ingredient in anterior chamber tissue and/or posterior chamber tissue of the eye and in the method for improving exposure of the active ingredient in anterior chamber tissue and/or posterior chamber tissue of the eye of the present disclosure described above may include, but is not limited to, water, saline, or any combination thereof.

In addition, based on the foregoing, the present disclosure further provides a composite formulation. The composite formulation described above may include an active ingredient, a delivery composition, and a pharmaceutically acceptable solvent for dissolving the active ingredient and the delivery composition to form a solution. The solution serves as the composite formulation, but is not limited thereto. In one embodiment, the composite formulation described above is an eye drop.

The relevant descriptions of the active ingredient in the composite formulation of the present disclosure may be referred to all relevant descriptions regarding the active ingredient in the foregoing descriptions relating to the eye drop platform for delivering the active ingredient to anterior chamber tissue and/or posterior chamber tissue of the eye of the present disclosure, and are not repeated herein.

In one embodiment, a concentration of the active ingredient in the solution of the composite formulation described above may range from about 0.05 to 500 mg/mL, for example, about 0.1 to 450 mg/mL, about 0.25 to 400 mg/mL, about 0.5 to 350 mg/mL, about 1 to 300 mg/mL, about 5 to 250 mg/mL, about 10 to 200 mg/mL, about 15 to 150 mg/mL, about 20 to 100 mg/mL, about 0.05 mg/mL, about 0.1 mg/mL, about 0.25 mg/mL, about 0.5 mg/mL, about 0.75 mg/mL, about 1 mg/mL, about 2 mg/mL, about 3 mg/mL, about 4 mg/mL, about 5 mg/mL, about 6 mg/mL, about 7 mg/mL, about 8 mg/mL, about 9 mg/mL, about 10 mg/mL, about 11 mg/mL, about 12 mg/mL, about 13 mg/mL, about 14 mg/mL, about 15 mg/mL, about 17 mg/mL, about 18 mg/mL, about 19 mg/mL, about 20 mg/mL, about 30 mg/mL, about 40 mg/mL, about 50 mg/mL, about 60 mg/mL, about 70 mg/mL, about 80 mg/mL, about 90 mg/mL, about 100 mg/mL, about 150 mg/mL, about 200 mg/mL, about 250 mg/mL, about 300 mg/mL, about 350 mg/mL, about 400 mg/mL, about 450 mg/mL, and about 500 mg/mL, but are not limited thereto.

In a specific embodiment, the active ingredient in the composite formulation of the present disclosure described above is sodium phenylbutyrate, and a concentration thereof in the solution may range from about 0.05 to 150 mg/mL, but is not limited thereto.

In another specific embodiment, the active ingredient in the composite formulation of the present disclosure described above may be metformin HCl, and a concentration thereof in the solution may range from about 200 to 300 mg/mL, but is not limited thereto.

In yet another specific embodiment, the active ingredient in the composite formulation of the present disclosure described above is dexmedetomidine HCl, and a concentration thereof in the solution may range from about 0.5 to 20 mg/mL, but is not limited thereto.

In a specific embodiment, the active ingredient in the composite formulation of the present disclosure described above is iptacopan, and a concentration thereof in the solution may range from about 1 to 25 mg/mL, but is not limited thereto.

In a specific embodiment, the active ingredient in the composite formulation of the present disclosure described above is tauro-ursodesoxy cholic acid (TUDCA), and a concentration thereof in the solution may range from about 5 to 50 mg/mL, but is not limited thereto.

Further, in a specific embodiment, the active ingredient in the composite formulation of the present disclosure described above is nafamostat mesylate, and a concentration thereof in the solution may range from about 10 to 150 mg/mL, but is not limited thereto.

In addition, in another specific embodiment, the active ingredient in the composite formulation of the present disclosure described above is (S)-N-(4-(1H-pyrazol-4-yl)phenyl)-3-amino-2-phenylpropanamide dihydrochloride (ITRI-E-(S)4046), and a concentration thereof in the solution may range from about 1 to 25 mg/mL, but is not limited thereto.

The delivery composition in the composite formulation of the present disclosure described above may be the same as the delivery composition in the eye drop platform for delivering an active ingredient to anterior chamber tissue and/or posterior chamber tissue of the eye of the present disclosure described above. Accordingly, all relevant descriptions of the delivery composition in the composite formulation and all relevant descriptions of the components thereof may likewise be referred to the foregoing paragraphs relating to the delivery composition and the components thereof in the eye drop platform for delivering an active ingredient to anterior chamber tissue and/or posterior chamber tissue of the eye of the present disclosure, and are not repeated herein.

Further, the pharmaceutically acceptable solvent in the composite formulation of the present disclosure described above may include, but is not limited to, water, saline, or any combination thereof.

In addition, based on the foregoing, the present disclosure further provides a use of any composite formulation described above in a preparation of an eye drop for treating and/or preventing an anterior chamber disease and/or a posterior chamber disease of an eye.

Similarly, based on the foregoing, the present disclosure further provides an eye drop for treating and/or preventing an anterior chamber disease and/or a posterior chamber disease of an eye. The eye drop may include, but is not limited to, any composite formulation described above.

Similarly, based on the foregoing, the present disclosure further provides a method for treating and/or preventing an anterior chamber disease and/or a posterior chamber disease of an eye. The method may include, but is not limited to, administering any composite formulation of the present disclosure described above or any eye drop for treating and/or preventing an anterior chamber disease and/or a posterior chamber disease of the eye of the present disclosure described above to a subject in need thereof.

The anterior chamber disease may include, but is not limited to, glaucoma, anterior uveitis, cataracts, dry eye, or any combination thereof.

The posterior chamber disease may include, but is not limited to, retinopathy. The retinopathy may include, but is not limited to, macular degeneration, macular edema, diabetic retinopathy, or any combination thereof. The macular degeneration may include, but is not limited to, age-related macular degeneration (AMD), pathologic myopia-related macular degeneration, or any combination thereof. The macular edema may include, but is not limited to, diabetic macular edema, but is not limited thereto.

Further, the present disclosure further provides a use of a composite formulation in a preparation of an eye drop for treating and/or preventing age-related macular degeneration. The composite formulation described herein may be any composite formulation of the present disclosure described above.

Similarly, based on the foregoing, the present disclosure further provides an eye drop for treating and/or preventing age-related macular degeneration. The eye drop of the present disclosure may include, but is not limited to, any composite formulation of the present disclosure described above.

Similarly, based on the foregoing, the present disclosure further provides a method for treating and/or preventing age-related macular degeneration. The method may include, but is not limited to, administering any composite formulation of the present disclosure described above or any eye drop for treating and/or preventing an anterior chamber disease and/or a posterior chamber disease of the eye of the present disclosure described above to a subject in need thereof.

In a specific embodiment, for the use of the composite formulation in the preparation of the eye drop for treating and/or preventing age-related macular degeneration of the present disclosure described above, the eye drop for treating and/or preventing age-related macular degeneration of the present disclosure described above, and the method for treating and/or preventing age-related macular degeneration of the present disclosure described above, the active ingredient in the composite formulation of the present disclosure described above is sodium phenylbutyrate; the cyclodextrin and/or the derivative thereof of the delivery composition in the composite formulation of the present disclosure described above is (2-hydroxypropyl)-β-cyclodextrin and/or (2-hydroxypropyl)-γ-cyclodextrin. A concentration of sodium phenylbutyrate in the solution may range from about 0.05 to 150 mg/mL, a concentration of (2-hydroxypropyl)-β-cyclodextrin and/or (2-hydroxypropyl)-γ-cyclodextrin in the solution may range from about 5 to 500 mg/mL, and a concentration of caffeine in the solution may range from about 0.5 to 50 mg/mL. Further, the delivery composition in the composite formulation may further include a water-soluble polymer. The water-soluble polymer is hydroxypropyl methyl cellulose, and a concentration of hydroxypropyl methyl cellulose in the solution may range from about 0.5 to 20 mg/mL.

As used herein, the term "subject" may include, but is not limited to, a vertebrate. The vertebrate may include, but is not limited to, a fish, an amphibian, a reptile, a bird, or a mammal. Examples of the mammal may include, but are not limited to, a human, an ape, a monkey, a horse, a donkey, a llama, a dog, a cat, a rabbit, a guinea pig, a rat, and a mouse. In one embodiment, the subject is a human.

### Example

### A. Drugs for Testing Delivery Efficiency of the Eye Drop Platform

Various drugs used in the experiments of the following examples and their respective functions are listed in Table 1 below. All drugs shown in Table 1 are hydrophilic drugs having a water solubility greater than 1 mg/mL.

**Table 1**

| **Hydrophilic Drug** | **Drug Function** |
|---|---|
| Sodium phenylbutyrate (SPB) | Neuroprotection |
| Metformin HCl | AMP-activated protein kinase (AMPK) activator |
| Dexmedetomidine HCl | Neuroprotection |
| Iptacopan (LNP-023) | Complement inhibitor |
| Tauro-ursodesoxy cholic acid (TUDCA) | Neuroprotection |
| Nafamostat mesylate (FUT-175) | Complement inhibitor |
| (S)-N-(4-(1H-pyrazol-4-yl)phenyl)-3-amino-2-phenylpropanamide dihydrochloride (ITRI-E-(S)4046) | Rho-associated coiled-coil kinase inhibitor |

### B. Experiments

### 1. Example 1

**Effect of Eye Drops Having Different Contents of Hydroxypropylmethyl cellulose (HPMC) on Exposure of Sodium Phenylbutyrate (SPB) Delivered to Retinal Tissue in the Posterior Chamber.**

### 1-1. Experimental Method

### 1-1-1. Preparation of Eye Drops Comprising Sodium Phenylbutyrate

2-hydroxypropyl-β-cyclodextrin (HPβCD), caffeine, hydroxypropylmethyl cellulose (HPMC), and sodium phenylbutyrate (SPB) were formulated at room temperature to prepare eye drops containing sodium phenylbutyrate (SPB), according to the respective concentrations shown in Table 2 below. Detailed procedures are described as follows.

**Table 2**

| **Formulation Group** | **Sodium Phenylbutyrate (SPB) (mg/mL)** | **2-Hydroxypropyl-β-cyclodextrin (HPβCD) (mg/mL)** | **Caffeine (mg/mL)** | **Hydroxypropylmethyl Cellulose (HPMC) (mg/mL)** |
|---|---|---|---|---|
| **1** | 30 | 0 | 0 | 0 |
| **2** | 30 | 125 | 20 | 0 |
| **3** | 30 | 125 | 20 | 5 |

2-hydroxypropyl-β-cyclodextrin (HPβCD), caffeine, and hydroxypropylmethyl cellulose (HPMC) were dissolved in deionized water at room temperature to form a clear aqueous solution.

Subsequently, sodium phenylbutyrate (SPB) was added to the aforementioned clear aqueous solution and stirred until sodium phenylbutyrate (SPB) was completely dissolved. Thereafter, deionized water was added to adjust the solution to a predetermined volume, and the solution was filtered through a filter having a pore size of 0.22 µm to obtain eye drops containing sodium phenylbutyrate (SPB).

Finally, a concentration of sodium phenylbutyrate (SPB) in the obtained eye drops containing sodium phenylbutyrate (SPB) was analyzed by high-performance liquid chromatography (HPLC).

### 1-1-2. Method for Exposure Analysis Test of Posterior Chamber Tissue

Pigmented rabbits purchased from a rabbit farm were weighed and then restrained in a restraining device. A volume of 35 µL of the test eye drops was instilled into the conjunctival sac of both the left and right eyes of each rabbit. Subsequently, the eyelids were closed and gently massaged to allow the eye drops to uniformly wet the entire ocular surface.

At 1, 3, and 6 hours after administration, the animals were sacrificed using CO₂. Subsequently, eyeballs were collected and ocular tissue dissection was performed to obtain retinal tissue and choroidal tissue belonging to posterior chamber tissue. Thereafter, the retinal tissue and the choroidal tissue were separately mixed with phosphate buffered saline (PBS) and homogenized to obtain a retinal homogenate to be tested and a choroidal homogenate to be tested.

Finally, the aforementioned retinal homogenate to be tested and the choroidal homogenate to be tested were analyzed for drug content by liquid chromatography-tandem mass spectrometry (LC-MS/MS). The procedures for processing and analyzing the retinal or choroidal samples are described as follows.

### (a) Preparation of Standard Solutions

First, a standard mixture solution was prepared by mixing drug reference standards with dimethyl sulfoxide (DMSO), and working solutions for calibration standards and quality control samples were prepared. Subsequently, each working solution was mixed with blank (untreated) rabbit retinal homogenate or choroidal homogenate at predetermined ratios to obtain calibration standards and quality control samples at different concentrations.

### (b) Extraction of Retinal Samples and Choroidal Samples

The calibration standards, quality control samples, and retinal homogenate samples for analysis or choroidal homogenate samples for analysis were added into respective 1.5 mL centrifuge tubes. Thereafter, an acetonitrile solution containing an internal standard was added to the centrifuge tubes and uniformly mixed using a shaker. The mixtures were then centrifuged to collect supernatants (>90 µL), and the supernatants were transferred into HPLC vials and analyzed by LC/MS/MS.

### 1-2. Experimental Results

The experimental results are shown in Table 3.

| **Formulation Group** | **1** | **2** | **3** |
|---|---|---|---|
| **Tissue Analyzed** | **Retinal tissue** | **Retinal tissue** | **Retinal tissue** |
| **Drug** | **Sodium phenylbutyrate (SPB)** | **Sodium phenylbutyrate (SPB)** | **Sodium phenylbutyrate (SPB)** |
| **Time Point (hours)** | **Exposure Concentration (ng/g)** | **Exposure Concentration (ng/g)** | **Exposure Concentration (ng/g)** |
| 1 | 6.3±12.6 | 2635.4±393.7 | 2555.0±884.9 |
| 3 | 8.7±8.1 | 1779.3±530.9 | 3441.0±125.1 |

According to Tables 2 and 3, for retinal tissue, the eye drops of group 2 and group 3 achieved exposure concentrations of sodium phenylbutyrate (SPB) that were significantly higher than those achieved by the eye drops of group 1. In other words, when the eye drops contained 2-hydroxypropyl-β-cyclodextrin (HPβCD) and caffeine, the eye drops were able to effectively increase the exposure of sodium phenylbutyrate (SPB) in retinal tissue.

Furthermore, according to Tables 2 and 3, the eye drops of group 3 were able to continuously enhance the exposure of sodium phenylbutyrate (SPB) in retinal tissue at 3 hours after administration. In other words, in addition to 2-hydroxypropyl-β-cyclodextrin (HPβCD) and caffeine, when the eye drops further contained hydroxypropylmethyl cellulose (HPMC), persistence of exposure of sodium phenylbutyrate (SPB) in retinal tissue was effectively improved.

### 2. Example 2

### Effect of Eye Drops Having Different Contents of Caffeine on Exposure of Sodium Phenylbutyrate (SPB) Delivered to Retinal Tissue in the Posterior Chamber

### 2-1. Experimental Methods

### 2-1-1. Preparation of Eye Drops Containing Sodium Phenylbutyrate (SPB)

2-hydroxypropyl-β-cyclodextrin (HPβCD), caffeine, hydroxypropylmethyl cellulose (HPMC), and sodium phenylbutyrate (SPB) were formulated at room temperature to prepare eye drops containing sodium phenylbutyrate (SPB), according to the respective concentrations of each formulation shown in Table 4 below. The preparation method of the eye drops may be carried out with reference to those described in Example 1 above.

**Table 4**

| **Formulation Group** | **Sodium Phenylbutyrate (SPB) (mg/mL)** | **2-Hydroxypropyl-β-cyclodextrin ( HPβCD) (mg/mL)** | **Caffeine (mg/mL)** | **Hydroxypropylmethyl Cellulose (HPMC) (mg/mL)** |
|---|---|---|---|---|
| 1 | 30 | 0 | 0 | 0 |
| 2 | 30 | 125 | 0 | 5 |
| 3 | 30 | 125 | 5 | 5 |
| 4 | 30 | 125 | 20 | 5 |
| 5 | 30 | 125 | 40 | 5 |

### 2-1-2. Method for Exposure Analysis Test of Posterior Chamber Tissue

Similarly, the method for the exposure analysis test of posterior chamber tissue may also be carried out with reference to that described in Example 1 above.

### 2-2. Experimental Results

The experimental results are shown in Table 5 below.

**Table 5**

| **Formulatio n Group** | **1** | **2** | **3** | **4** | **5** |
|---|---|---|---|---|---|
| **Tissue analyzed** | **Retinal tissue** | **Retinal tissue** | **Retinal tissue** | **Retinal tissue** | **Retinal tissue** |
| **Drug** | **Sodium phenylbutyra te (SPB)** | **Sodium phenylbutyra te (SPB)** | **Sodium phenylbutyra te (SPB)** | **Sodium phenylbutyra te (SPB)** | **Sodium phenylbutyra te (SPB)** |
| **Time Point (hours)** | **Exposure Concentratio n (ng/g)** | **Exposure Concentratio n (ng/g)** | **Exposure Concentratio n (ng/g)** | **Exposure Concentratio n (ng/g)** | **Exposure Concentratio n (ng/g)** |
| 1 | 6.3±12.6 | 28.7±20.6 | 569.1±49.9 | 2555.0±884.9 | 3908.5±425.8 |
| 3 | 8.7±8.1 | 69.6±85.5 | 904.8±293.9 | 3441.0±125.1 | 3842.8±456.8 |

According to Tables 4 and 5, the eye drops of group 1 achieved a maximum exposure concentration of sodium phenylbutyrate (SPB) in the retinal tissue of less than 10 ng/g after administration. In other words, a solution containing solely sodium phenylbutyrate (SPB) exhibited limited exposure of sodium phenylbutyrate (SPB) in the retinal tissue.

In contrast, the eye drops of group 2 achieved a maximum exposure concentration of sodium phenylbutyrate (SPB) in the retinal tissue of up to 69.6 ng/g after administration. In other words, when the eye drops contained 2-hydroxypropyl-β-cyclodextrin (HPβCD), the exposure of sodium phenylbutyrate (SPB) in the retinal tissue was enhanced.

Furthermore, groups 3 to 5 demonstrated that when the eye drops, in addition to 2-hydroxypropyl-β-cyclodextrin (HPβCD), further contained caffeine at concentrations of 5-40 mg/mL, the exposure concentration of sodium phenylbutyrate (SPB) in the retinal tissue was significantly increased (904.8-3908.5 ng/g). Moreover, the concentration of caffeine exhibited a positive correlation with the exposure concentration of sodium phenylbutyrate (SPB) in the retinal tissue.

The above experimental data demonstrate that eye drops containing 2-hydroxypropyl-β-cyclodextrin (HPβCD) and caffeine are capable of increasing the exposure of sodium phenylbutyrate (SPB) in the retinal tissue.

### 3. Example 3

### Effect of Eye Drops Having Different Contents of 2-Hydroxypropyl-β-Cyclodextrin (HPβCD) on Exposure of Sodium Phenylbutyrate (SPB) Delivered to Retinal Tissue in the Posterior Chamber

### 3-1. Experimental Methods

### 3-1-1. Preparation of Eye Drops Containing Sodium Phenylbutyrate (SPB)

2-hydroxypropyl-β-cyclodextrin (HPβCD), caffeine, hydroxypropylmethyl cellulose (HPMC), and sodium phenylbutyrate (SPB) were formulated at room temperature to prepare eye drops containing sodium phenylbutyrate (SPB), according to the respective concentrations of each formulation shown in Table 6 below. The preparation method of the eye drops and the method for the exposure analysis test of posterior chamber tissue may be carried out with reference to those described in Example 1 above.

**Table 6**

| **Formulation Group** | **Sodium Phenylbutyrate (SPB) (mg/mL)** | **2-Hydroxypropyl-β-cyclodextrin ( HPβCD) (mg/mL)** | **Caffeine (mg/mL)** | **Hydroxypropyl methyl Cellulose (HPMC) (mg/mL)** |
|---|---|---|---|---|
| 1 | 30 | 0 | 0 | 0 |
| 2 | 30 | 60 | 20 | 5 |
| 3 | 30 | 125 | 20 | 5 |
| 4 | 30 | 400 | 20 | 5 |
| 5 | 40 | 30 | 20 | 5 |
| 6 | 60 | 30 | 20 | 5 |

### 3-1-2. Method for Exposure Analysis Test of Posterior Chamber Tissue

Similarly, the method for the exposure analysis test of posterior chamber tissue may also be carried out with reference to that described in Example 1 above.

### 3-2. Experimental Results

The experimental results are shown in Table 7 below.

**Table 7**

| Formulation Group | 1 | 2 | 3 | 4 | 5 | 6 |
|---|---|---|---|---|---|---|
| **Tissue Analyzed** | **Retinal tissue** | **Retinal tissue** | **Retinal tissue** | **Retinal tissue** | **Retinal tissue** | **Retinal tissue** |
| **Drug** | **Sodium phenylbut yrate (SPB)** | **Sodium phenylbut yrate (SPB)** | **Sodium phenylbut yrate (SPB)** | **Sodium phenylbut yrate (SPB)** | **Sodium phenylbut yrate (SPB)** | **Sodium phenylbut yrate (SPB)** |
| **Time Point (hours)** | **Exposure Concentra tion (ng/g)** | **Exposure Concentra tion (ng/g)** | **Exposure Concentra tion (ng/g)** | **Exposure Concentra tion (ng/g)** | **Exposure Concentra tion (ng/g)** | **Exposure Concentra tion (ng/g)** |
| 1 | 6.3±12.6 | 4321.3±45 0.0 | 2555.0±88 4.9 | 4385.0±12 18.1 | 5486.5±33 7.4 | 5349.5±79 2.3 |
| 3 | 8.7±8.1 | 4208.8±12 51.1 | 3441.0±12 5.1 | 4225.0±40 8.8 | 2577.0±15 5.3 | 4208.5±14 34.0 |

According to Tables 6 and 7, the eye drops of groups 2 to 4 achieved maximum exposure concentrations of sodium phenylbutyrate (SPB) in the retinal tissue after administration that were significantly higher than those achieved by the eye drops of group 1 after administration. Specifically, when the concentration of sodium phenylbutyrate (SPB) in the eye drops was fixed at 30 mg/mL, the concentration of caffeine was fixed at 20 mg/mL, and the concentration of 2-hydroxypropyl-β-cyclodextrin (HPβCD) was adjusted to 60-400 mg/mL, the maximum concentration of sodium phenylbutyrate (SPB) in the retinal tissue after administration reached 3441.0-4385.0 ng/g. These concentrations are all significantly higher than the exposure concentration of sodium phenylbutyrate (SPB) in the retinal tissue achieved by a solution containing solely sodium phenylbutyrate (SPB).

Furthermore, the administration results of the eye drops of groups 5 and 6 showed that when the concentration of sodium phenylbutyrate (SPB) in the eye drops was fixed at 40 mg/mL, the concentration of caffeine was fixed at 20 mg/mL, and the concentration of 2-hydroxypropyl-β-cyclodextrin (HPβCD) was adjusted to 30-60 mg/mL, the maximum concentration of sodium phenylbutyrate (SPB) in the retinal tissue after administration reached 5349.5-5486.5 ng/g.

### 4. Example 4

### Effect of Eye Drops Containing Different Types of Cyclodextrins on Exposure of Sodium Phenylbutyrate (SPB) Delivered to Retinal Tissue in the Posterior Chamber

### 4-1. Experimental Methods

### 4-1-1. Preparation of Eye Drops Containing Sodium Phenylbutyrate (SPB)

Different types of cyclodextrins, caffeine, hydroxypropylmethyl cellulose (HPMC), and sodium phenylbutyrate (SPB) were formulated at room temperature to prepare eye drops containing sodium phenylbutyrate (SPB), according to the respective concentrations of each formulation shown in Table 8 below. The preparation method of the eye drops may be carried out with reference to that described in Example 1 above.

**Table 8**

| **Formulatio n Group** | **Sodium Phenylbuty rate (SPB) (mg/mL)** | **2- Hydroxypr opyl-β-cyclodextri n (HPβCD) (mg/mL)** | **2- Hydroxypr opyl)-γ-cyclodextri n (HPγCD) (mg/mL)** | **Caffeine (mg/mL)** | **Hydroxypr opylmethyl Cellulose (HPMC) (mg/mL)** |
|---|---|---|---|---|---|
| 1 | 30 | 0 | 0 | 0 | 0 |
| 2 | 30 | 125 | 0 | 20 | 5 |
| 3 | 30 | 0 | 125 | 20 | 5 |

### 4-1-2. Method for Exposure Analysis Test of Posterior Chamber Tissue

Similarly, the method for the exposure analysis test of posterior chamber tissue may also be carried out with reference to that described in Example 1 above.

### 4-2. Experimental Results

The experimental results are shown in Table 9 below.

**Table 9**

| **Formulation Group** | **1** | **2** | **3** |
|---|---|---|---|
| **Tissue Analyzed** | **Retinal tissue** | **Retinal tissue** | **Retinal tissue** |
| **Drug** | **Sodium phenylbutyrate (SPB)** | **Sodium phenylbutyrate (SPB)** | **Sodium phenylbutyrate (SPB)** |
| **Time Point (hours)** | **Exposure Concentration (ng/g)** | **Exposure Concentration (ng/g)** | **Exposure Concentration (ng/g)** |
| 1 | 6.3±12.6 | 2555.0±884.9 | 1725.5±538.2 |
| 3 | 8.7±8.1 | 3441.0±125.1 | 3447.5±1157.9 |

According to Tables 8 and 9, the eye drops of groups 2 and 3 achieved maximum exposure concentrations of sodium phenylbutyrate (SPB) in the retinal tissue after administration that were significantly higher than those achieved by the eye drops of group 1 after administration. Specifically, when the concentration of sodium phenylbutyrate (SPB) in the eye drops was fixed at 30 mg/mL and the concentration of caffeine was fixed at 20 mg/mL, regardless of whether 2-hydroxypropyl-β-cyclodextrin (HPβCD) or 2-hydroxypropyl-γ-cyclodextrin (HPγCD) was used, the maximum concentration of sodium phenylbutyrate (SPB) in the retinal tissue after administration reached 3441.0-3447.5 ng/g. These concentrations are all significantly higher than the exposure concentration of sodium phenylbutyrate (SPB) in the retinal tissue achieved by a solution containing solely sodium phenylbutyrate (SPB).

The above data demonstrate that eye drops formulated with 2-hydroxypropyl-β-cyclodextrin (HPβCD) or 2-hydroxypropyl-γ-cyclodextrin (HPγCD) in combination with caffeine are capable of enhancing the exposure of sodium phenylbutyrate (SPB) in retinal tissue.

### 5. Example 5

### Effect of Eye Drops Having Specific Contents of Cyclodextrin and Caffeine on Exposure of Metformin HCl Delivered to Choroidal Tissue in the Posterior Chamber

### 5-1. Experimental Methods

### 5-1-1. Preparation of Eye Drops Containing Metformin HCl

2-hydroxypropyl-β-cyclodextrin (HPβCD), caffeine, hydroxypropylmethyl cellulose (HPMC), and metformin HCl were formulated at room temperature to prepare eye drops containing metformin HCl, according to the respective concentrations of each formulation shown in Table 10 below. With respect to the preparation method of the eye drops, except that sodium phenylbutyrate (SPB) was replaced with metformin HCl, all other procedures were the same as those described in Example 1 above.

**Table 10**

| **Formulation Group** | **Metformin HCl (mg/mL)** | **2-Hydroxypropyl-β-cyclodextrin ( HPβCD) (mg/mL)** | **Caffeine (mg/mL)** | **Hydroxypropyl methyl Cellulose (HPMC) (mg/mL)** |
|---|---|---|---|---|
| Metformin HCl solution | 250 | 0 | 0 | 0 |
| Metformin HCl-cyclodextrin solution | 250 | 400 | 20 | 5 |

### 5-1-2. Method for Exposure Analysis Test of Posterior Chamber Tissue

With respect to the method for the exposure analysis test of posterior chamber tissue, except that the time points for animal administration were adjusted to 0.5, 1, and 3 hours, all other procedures may also be the same as those described in Example 1 above.

### 5-2. Experimental Results

The experimental results are shown in Table 11 below.

**Table 11**

| **Formulation Group** | **1** | **2** |
|---|---|---|
| **Tissue Analyzed** | **Choroidal tissue** | **Choroidal tissue** |
| **Drug** | **Metformin HCl** | **Metformin HCl** |
| **Time Point (hours)** | **Exposure Concentration (ng/g)** | **Exposure Concentration (ng/g)** |
| 0.5 | 4683.8±820.9 | 20150.0±8393.8 |
| 1 | 2026.7±754.2 | 26437.5±6950.0 |
| 3 | 3306.3±2262.0 | 22362.5±10587.4 |

According to Tables 10 and 11, under conditions in which the eye drops containing metformin HCl further included 400 mg/mL of 2-hydroxypropyl-β-cyclodextrin (HPβCD) and 20 mg/mL of caffeine, the maximum concentration of metformin HCl in the choroidal tissue after administration reached 26,437.5 ng/g, which was significantly higher than that achieved by eye drops consisting solely of a metformin HCl solution.

The above experimental data demonstrate that eye drops containing 2-hydroxypropyl-β-cyclodextrin (HPβCD) and caffeine are capable of increasing the exposure of metformin HCl in choroidal tissue.

### 6. Example 6

### Effect of Eye Drops Having Specific Contents of Cyclodextrin and Caffeine on Exposure of Dexmedetomidine HCl Delivered to Retinal Tissue and Choroidal Tissue in the Posterior Chamber

### 6-1. Experimental Methods

### 6-1-1. Preparation of Eye Drops Containing Dexmedetomidine HCl

2-hydroxypropyl-β-cyclodextrin (HPβCD), caffeine, hydroxypropylmethyl cellulose (HPMC), and dexmedetomidine HCl were formulated at room temperature to prepare eye drops containing dexmedetomidine HCl, according to the respective concentrations of each formulation shown in Table 10 below. With respect to the preparation method of the eye drops, except that sodium phenylbutyrate (SPB) was replaced with dexmedetomidine HCl, all other procedures were the same as those described in Example 1 above.

**Table 12**

| **Formulation Group** | **Dexmedetomidine HCl (mg/mL)** | **2-Hydroxypropyl -β-cyclodextrin ( HPβCD) (mg/mL)** | **Caffeine (mg/mL)** | **Hydroxypropylm ethyl Cellulose (HPMC) (mg/mL)** |
|---|---|---|---|---|
| Dexmedetomidine HCl- cyclodextrin solution | 8 | 400 | 20 | 5 |

### 6-1-2. Method for Exposure Analysis Test of Posterior Chamber Tissue

With respect to the method for the exposure analysis test of posterior chamber tissue, except that the time points for animal administration were adjusted to 0.5 and 3 hours, all other procedures may also be carried out with reference to those described in Example 1 above.

### 6-2. Experimental Results

The experimental results are shown in Table 13 below.

**Table 13**

| **Formulation Group** | **Dexmedetomidine HCl-cyclodextrin solution** | |
|---|---|---|
| **Tissue Analyzed** | **Retinal tissue** | **Choroidal tissue** |
| **Drug** | **Dexmedetomidine HCl** | **Dexmedetomidine HCl** |
| **Time Point (hours)** | **Exposure Concentration (ng/g)** | **Exposure Concentration (ng/g)** |
| 0.5 | 290.5±86.4 | 1538.8±222.5 |
| 3 | 94.2±40.9 | 2711.7±1472.7 |

According to Tables 12 and 13, under conditions in which the eye drops containing dexmedetomidine HCl further included 400 mg/mL of 2-hydroxypropyl-β-cyclodextrin (HPβCD) and 20 mg/mL of caffeine, the maximum concentration of dexmedetomidine HCl in the retinal tissue after administration reached 290.5 ng/g, and the maximum concentration of dexmedetomidine HCl in the choroidal tissue after administration reached 2711.7 ng/g.

The above experimental data demonstrate that eye drops containing 2-hydroxypropyl-β-cyclodextrin (HPβCD) and caffeine are capable of effectively delivering dexmedetomidine HCl to posterior chamber tissue.

### 7. Example 7

### Effect of Eye Drops Having Specific Contents of Cyclodextrin and Caffeine on Exposure of Iptacopan Delivered to Retinal Tissue and Choroidal Tissue in the Posterior Chamber

### 7-1. Experimental Methods

### 7-1-1. Preparation of eye drop formulations containing iptacopan

2-hydroxypropyl-β-cyclodextrin (HPβCD), caffeine, hydroxypropylmethyl cellulose (HPMC), and iptacopan were formulated at room temperature to prepare eye drops containing Iptacopan, according to the respective concentrations of each formulation shown in Table 14 below. With respect to the preparation method of the eye drops, except that sodium phenylbutyrate (SPB) was replaced with iptacopan, all other procedures were the same as those described in Example 1 above.

**Table 14**

| **Formulation Group** | **Iptacopan (mg/mL)** | **2-Hydroxypropyl-β-cyclodextrin (HPβCD) (mg/mL)** | **Caffeine (mg/mL)** | **Hydroxypropylmethyl Cellulose (HPMC) (mg/mL)** |
|---|---|---|---|---|
| iptacopan-cyclodextrin Solution | 10 | 400 | 20 | 5 |

### 7-1-2. Method for Exposure Analysis Test of Posterior Chamber Tissues

With respect to the method for analyzing the exposure of posterior chamber tissues, except that the time points for animal administration were adjusted to 0.5 and 3 hours, all other procedures were also the same as those described in Example 1 above.

### 7-2. Experimental Results

The experimental results are shown in Table 15 below.

**Table 15**

| **Formulation Group** | **Iptacopan-cyclodextrin Solution** | |
|---|---|---|
| **Tissue Analyzed** | **Retinal tissue** | **Choroidal tissue** |
| **Drug** | **Iptacopan** | **Iptacopan** |
| **Time Point (hours)** | **Exposure Concentration (ng/g)** | **Exposure Concentration (ng/g)** |
| 0.5 | 86.6±24.8 | 268.0±87.8 |
| 3 | 53.9±20.1 | 208.1±101.8 |

According to Tables 14 and 15, when the eye drops containing iptacopan comprised 400 mg/mL of 2-hydroxypropyl-β-cyclodextrin (HPβCD) and 20 mg/mL of caffeine, the maximum concentration of iptacopan in the retinal tissue after administration reached 86.6 ng/g, and the maximum concentration in the choroidal tissue reached 268.0 ng/g.

The above experimental data demonstrate that eye drops containing 2-hydroxypropyl-β-cyclodextrin (HPβCD) and caffeine can effectively deliver Iptacopan to posterior chamber tissues.

### 8. Example 8

### Effect of Eye Drops Having Specific Contents of Cyclodextrin and Caffeine on Exposure of Tauro-Ursodesoxy Cholic acid (TUDCA) Delivered to Retinal Tissue and Choroidal Tissue in the Posterior Chamber

### 8-1. Experimental Methods

### 8-1-1. Preparation of Eye Drops Containing Sodium Phenylbutyrate

2-hydroxypropyl-β-cyclodextrin (HPβCD), caffeine, hydroxypropylmethyl cellulose (HPMC), and tauro-ursodesoxy cholic acid (TUDCA) were formulated at room temperature to prepare eye drops containing tauro-ursodesoxy cholic acid (TUDCA), according to the respective concentrations of each formulation shown in Table 18 below. With respect to the preparation method of the eye drops, except that sodium phenylbutyrate (SPB) was replaced with tauro-ursodesoxy cholic acid (TUDCA), all other procedures were the same as those described in Example 1 above.

**Table 18**

| **Formulation Group** | **Tauro-Ursodesoxy Cholic acid (TUDCA) (mg/mL)** | **2-Hydroxypropyl-β-cyclodextrin (HPβCD) (mg/mL)** | **Caffeine (mg/mL)** | **Hydroxypropylmethyl Cellulose (HPMC) (mg/mL)** |
|---|---|---|---|---|
| Tauro-ursodesoxy Cholic acid (TUDCA)-Cyclodextrin Solution | 20 | 400 | 20 | 5 |

### 8-1-2. Method for Exposure Analysis Test of Posterior Chamber Tissue

With respect to the method for analyzing the exposure of posterior chamber tissues, except that the time points for animal administration were adjusted to 0.5 and 3 hours, all other procedures were the same as those described in Example 1 above.

### 8-2. Experimental Results

The experimental results are shown in Table 17 below.

**Table 17**

| **Formulation Group** | **Tauro-ursodesoxy cholic acid (TUDCA)-cyclodextrin solution** | |
|---|---|---|
| **Tissue Analyzed** | **Retinal tissue** | **Choroidal tissue** |
| **Drug** | **Tauro-ursodesoxy cholic acid (TUDCA)** | **Tauro-ursodesoxy cholic acid (TUDCA)** |
| **Time Point (hours)** | **Exposure Concentration (ng/g)** | **Exposure Concentration (ng/g)** |
| 0.5 | 43.3±39.2 | 38.7±29.0 |
| 3 | 73.0±66.8 | 25.3±5.4 |

According to Tables 16 and 17, when the eye drops containing tauro-ursodesoxy cholic acid (TUDCA) comprised 400 mg/mL of 2-hydroxypropyl-β-cyclodextrin (HPβCD) and 20 mg/mL of caffeine, the maximum concentration of tauro-ursodesoxy cholic acid (TUDCA) in the retinal tissue after administration reached 73.0 ng/g, and the maximum concentration in the choroidal tissue reached 38.7 ng/g.

The above experimental data demonstrate that eye drops containing 2-hydroxypropyl-β-cyclodextrin (HPβCD) and caffeine can effectively deliver tauro-ursodesoxy cholic acid (TUDCA) to posterior chamber tissues.

### 9. Example 9

### Effect of Eye Drops Having Specific Contents of Cyclodextrin and Caffeine on Exposure of Nafamostat Mesylate (FUT-175) Delivered to Retinal Tissue and Choroidal Tissue in the Posterior Chamber

### 9-1. Experimental Methods

### 9-1-1. Preparation of Eye Drops Containing Nafamostat Mesylate (FUT-175)

2-hydroxypropyl-β-cyclodextrin (HPβCD), caffeine, hydroxypropylmethyl cellulose (HPMC), and nafamostat mesylate (FUT-175) were formulated at room temperature to prepare eye drops containing nafamostat mesylate (FUT-175), according to the respective concentrations of each formulation shown in Table 18 below. With respect to the preparation method of the eye drops, except that sodium phenylbutyrate (SPB) was replaced with nafamostat mesylate (FUT-175), all other procedures were the same as those described in Example 1 above.

**Table 18**

| **Formulation Group** | **Nafamostat Mesylate (FUT-175) (mg/mL)** | **2-Hydroxypropyl-β-cyclodextrin (HPβCD) (mg/mL)** | **Caffeine (mg/mL)** | **Hydroxypropylme thyl Cellulose (HPMC) (mg/mL)** |
|---|---|---|---|---|
| 30 mg/mL Nafamostat Mesylate (FUT-175) Cyclodextrin Solution | 30 | 400 | 20 | 5 |
| 100 mg/mL Nafamostat Mesylate (FUT-175)-Cyclodextrin solution | 100 | 400 | 20 | 5 |

### 9-1-2. Method for Exposure Analysis Test of Posterior Chamber Tissue:

With respect to the method for analyzing the exposure of posterior chamber tissue, except that the time points for animal administration were adjusted to 0.5, 1, and 3 hours, all other procedures were the same as those described in Example 1 above.

### 9-2. Experimental results

The experimental results are shown in Table 19 below.

**Table 19**

| Formulation **Group** | **30 mg/mL Nafamostat Mesylate** (FUT-175)-Cyclodextrin **Solution** | | **100 mg/mL Nafamostat** Mesylate (FUT-175)-**Cyclodextrin Solution** | |
|---|---|---|---|---|
| **Tissue Analyzed** | **Retinal tissue** | **Choroidal tissue** | **Retinal tissue** | **Choroidal tissue** |
| **Drug** | **Nafamostat mesylate (FUT-175)** | **Nafamostat mesylate (FUT-175)** | **Nafamostat mesylate (FUT-175)** | **Nafamostat mesylate (FUT-175)** |
| **Time Point (hours)** | **Exposure Concentration (ng/g)** | **Exposure Concentration (ng/g)** | **Exposure Concentration (ng/g)** | **Exposure Concentration (ng/g)** |
| 0.5 | 24.8±10.0 | 396.1±117.9 | 117.5±32.7 | 1165.0±221.9 |
| 1 | 60.0±32.3 | 418.5±189.6 | 214.5±53.3 | 813.8±296.1 |
| 3 | 58.4±21.6 | 260.1±136.4 | 261.9±43.2 | 938.8±372.0 |

According to Tables 18 and 19, when the eye drops containing 30 mg/mL of nafamostat mesylate (FUT-175) comprised 400 mg/mL of 2-hydroxypropyl-β-cyclodextrin (HPβCD) and 20 mg/mL of caffeine, the maximum concentration of nafamostat mesylate (FUT-175) in the retinal tissue after administration reached 60.0 ng/g, and the maximum concentration in the choroidal tissue reached 418.5 ng/g.

When the eye drops containing 100 mg/mL of nafamostat mesylate (FUT-175) comprised 400 mg/mL of 2-hydroxypropyl-β-cyclodextrin (HPβCD) and 20 mg/mL of caffeine, the maximum concentration of nafamostat mesylate (FUT-175) in the retinal tissue after administration reached 261.9 ng/g, and the maximum concentration in the choroidal tissue reached 1165.0 ng/g.

The above experimental data demonstrate that eye drops containing 2-hydroxypropyl-β-cyclodextrin (HPβCD) and caffeine can effectively deliver nafamostat mesylate (FUT-175) to posterior chamber tissues.

### 10. Example 10

### Effect of Eye Drops Having Specific Contents of Cyclodextrin and Caffeine on Exposure of (S)-N-(4-(1H-pyrazol-4-yl)phenyl)-3-amino-2-phenylpropanamide dihydrochloride (ITRI-E-(S)4046) Delivered to Retinal Tissue and Choroidal Tissue in the Posterior Chamber

### 10-1. Experimental Methods

### 10-1-1. Preparation of Eye Drops Containing (S)-N-(4-(1H-pyrazol-4-yl)phenyl)-3-amino-2-phenylpropanamide dihydrochloride (ITRI-E-(S)4046)

2-hydroxypropyl-β-cyclodextrin (HPβCD), caffeine, hydroxypropylmethyl cellulose (HPMC), and (S)-N-(4-(1H-pyrazol-4-yl)phenyl)-3-amino-2-phenylpropanamide dihydrochloride (ITRI-E-(S)4046) were formulated at room temperature to prepare eye drops containing (S)-N-(4-(1H-pyrazol-4-yl)phenyl)-3-amino-2-phenylpropanamide dihydrochloride (ITRI-E-(S)4046), according to the respective concentrations of each formulation shown in Table 20 below. With respect to the preparation method of the eye drops, except that sodium phenylbutyrate (SPB) was replaced with (S)-N-(4-(1H-pyrazol-4-yl)phenyl)-3-amino-2-phenylpropanamide dihydrochloride (ITRI-E-(S)4046), all other procedures were the same as those described in Example 1 above.

**Table 20**

| **Formulation Group** | **(S)-N-(4-(1H-pyrazol-4-yl)phenyl)-3-amino-2-phenylpropana mide dihydrochlorid e (ITRI-E-(S)4046) (mg/mL)** | **2-Hydroxypropyl-β-cyclodextrin (HPβCD) (mg/mL)** | **Caffeine (mg/mL)** | **Hydroxypropy lmethyl Cellulose (HPMC) (mg/mL)** |
|---|---|---|---|---|
| (S)-N-(4-(1H-pyrazol-4-yl)phenyl)-3-amino-2-phenylpropanamid e Dihydrochloride (ITRI-E-(S)4046)-Cyclodextrin Solution | 8 | 400 | 20 | 5 |

### 10-1-2. Method for Exposure Analysis Test of Posterior Chamber Tissue

With respect to the method for exposure analysis test of posterior chamber tissue, except that the time points for animal administration were adjusted to 0.5 and 3 hours, all other procedures were the same as those described in Example 1 above.

### 10-2. Experimental Results

The experimental results are shown in Table 21 below.

**Table 21**

| **Formulation Group** | **(S)-N-(4-(1H-pyrazol-4-yl)phenyl)-3-amino-2-phenylpropanamide Dihydrochloride (ITRI-E-(S)4046)-Cyclodextrin Solution** | |
|---|---|---|
| **Tissue Analyzed** | **Retinal tissue** | **Choroidal tissue** |
| **Drug** | **(S)-N-(4-(1H-pyrazol-4-yl)phenyl)-3-amino-2-phenylpropanamide dihydrochloride (ITRI-E-(S)4046)** | **(S)-N-(4-(1H-pyrazol-4-yl)phenyl)-3-amino-2-phenylpropanamide dihydrochloride (ITRI-E-(S)4046)** |
| **Time Point (hours)** | **Exposure Concentration (ng/g)** | **Exposure Concentration (ng/g)** |
| 0.5 | 70.3±3.5 | 78.3±11.7 |
| 3 | 71.5±12.4 | 76.8±16.0 |

According to Tables 20 and 21, when the eye drops containing (S)-N-(4-(1H-pyrazol-4-yl)phenyl)-3-amino-2-phenylpropanamide dihydrochloride (ITRI-E-(S)4046) comprised 400 mg/mL of 2-hydroxypropyl-β-cyclodextrin (HPβCD) and 20 mg/mL of caffeine, the maximum concentration of (S)-N-(4-(1H-pyrazol-4-yl)phenyl)-3-amino-2-phenylpropanamide dihydrochloride (ITRI-E-(S)4046) in the retinal tissue after administration reached 71.5 ng/g, and the maximum concentration in the choroidal tissue reached 78.3 ng/g.

The above experimental data demonstrate that eye drops containing 2-hydroxypropyl-β-cyclodextrin (HPβCD) and caffeine can effectively deliver (S)-N-(4-(1H-pyrazol-4-yl)phenyl)-3-amino-2-phenylpropanamide dihydrochloride (ITRI-E-(S)4046) to posterior chamber tissues.

### 11. Example 11

### Verification of the Therapeutic Effect of Eye Drops Containing Sodium Phenylbutyrate (SPB) in an Animal Disease Model Induced by Sodium Iodate (NaIO₃)

### 11-1. Experimental Methods

### 11-1-1. Preparation of Eye Drops Containing Sodium Phenylbutyrate (SPB)

2-hydroxypropyl-β-cyclodextrin (HPβCD), caffeine, hydroxypropylmethyl cellulose (HPMC), and sodium phenylbutyrate (SPB) were formulated at room temperature to prepare eye drops containing sodium phenylbutyrate (SPB), according to the respective concentrations of each formulation shown in Table 22 below. With respect to the preparation method of the eye drops, the procedures may be carried out with reference to those described in Example 1 above.

**Table 22**

| **Formulation Group** | **Sodium Phenylbutyrate (SPB) (mg/mL)** | **2-Hydroxypropyl-β-cyclodextrin (HPβCD) (mg/mL)** | **Caffeine (mg/mL)** | **Hydroxypropylmethyl Cellulose (HPMC) (mg/mL)** |
|---|---|---|---|---|
| **1** | 1 | 125 | 20 | 5 |
| **2** | 10 | 125 | 20 | 5 |
| **3** | 100 | 125 | 20 | 5 |

### 11-1-2. Construction and Analysis of a NaIO₃-Induced Animal Disease Model

A sodium iodate (NaIO₃)-induced animal disease model can serve as an animal model for age-related macular degeneration (AMD).

Pigmented rabbits were sedated and anesthetized by intramuscular injection of a mixed solution of Rompun and Zoletil 50, followed by ocular anesthesia and pupil dilation using 0.5% Alcaine and Mydrin-P eye drops in sequence.

Next, refer to FIG. 1A. FIG. 1A illustrates an experimental procedure of the NaIO₃-induced animal disease model. First, pigmented rabbits were administered a single intravitreal injection of 50 µL of an 8 mg/mL NaIO₃ solution using a 29 G needle to induce fundus retinal degeneration, while an antibiotic ointment was simultaneously applied to reduce the risk of infection associated with intravitreal injection. During the NaIO₃-induced retinal degeneration period, the previously prepared eye drops containing sodium phenylbutyrate (SPB) were administered four times daily. After 7 consecutive days of administration, the experimental animals were sacrificed and eyeball samples were collected.

Subsequently, ocular tissues were embedded in paraffin and sectioned, and the sections were subjected to hematoxylin and eosin (H&E) staining. Finally, based on the H&E staining results, changes in the thickness of the outer nuclear layer (ONL) of the retina, which is a region containing the nuclei of retinal photoreceptor cells composed of rods and cones, in rabbit retinal tissue sections were evaluated as an indicator of therapeutic efficacy.

### 11-2. Experimental Results

The experimental results are shown in FIGs. 1B and 1C.

According to FIGs. 1B and 1C, sodium phenylbutyrate (SPB) was effectively delivered to the retinal tissue by the prepared eye drops containing sodium phenylbutyrate (SPB).

In addition, compared with the untreated group, the prepared eye drops containing sodium phenylbutyrate (SPB) effectively preserved the thickness of the outer nuclear layer (ONL) of the retina under NaIO₃-induced retinal degeneration. In other words, the prepared eye drops containing sodium phenylbutyrate (SPB) exhibited a protective effect on the outer nuclear layer of the retina.

Furthermore, the eye drops prepared with low, medium, and high doses of sodium phenylbutyrate (SPB) achieved improvement efficiencies for retinal degeneration of 8.92%, 42.84%, and 75.53%, respectively, indicating a dose-dependent therapeutic effect.

### 12. Example 12

### Verification of the Therapeutic Effect of Eye Drops Containing Sodium Phenylbutyrate (SPB) in an Animal Disease Model Induced by Hydroquinone (HQ)

### 12-1. Experimental Methods

### 12-1-1. Preparation of Eye Drops Containing Sodium Phenylbutyrate (SPB)

2-hydroxypropyl-β-cyclodextrin (HPβCD), caffeine, hydroxypropylmethyl cellulose (HPMC), and sodium phenylbutyrate (SPB) were formulated at room temperature to prepare eye drops containing sodium phenylbutyrate (SPB), according to the respective concentrations of each formulation shown in Table 23 below. With respect to the preparation method of the eye drops, the procedures may be carried out with reference to those described in Example 1 above.

**Table 23**

| **Formulation Group** | **Sodium Phenylbutyrate (SPB) (mg/mL)** | **2-Hydroxypropyl-β-cyclodextrin (HPβCD) (mg/mL)** | **Caffeine (mg/mL)** | **Hydroxypropylmethyl Cellulose (HPMC) (mg/mL)** |
|---|---|---|---|---|
| **1** | 10 | 125 | 20 | 5 |
| **2** | 30 | 125 | 20 | 5 |
| **3** | 60 | 125 | 20 | 5 |
| **4** | 100 | 125 | 20 | 5 |

### 12-1-2. Construction and Analysis of a Hydroquinone (HQ)-Induced Animal Disease Model

A hydroquinone (HQ)-induced animal disease model can serve as an animal model for age-related macular degeneration (AMD).

C57BL/6 mice were anesthetized by intraperitoneal injection of a mixed solution of Zoletil 50 and xylazine, followed by ocular anesthesia achieved by instilling 5 µL of Alcaine into both eyes of each mouse.

Next, refer to FIG. 2A. FIG. 2A illustrates an experimental procedure of the hydroquinone (HQ)-induced animal disease model.

First, C57BL/6 mice were administered a subconjunctival injection of 10 µL of a 75 mM hydroquinone (HQ) solution per eye using a ≥30 G needle, at an injection frequency of 3 times per week, to establish a disease animal model. After hydroquinone (HQ) induction and observation of lesion formation, the previously prepared eye drops containing sodium phenylbutyrate (SPB) were administered at a frequency of 4 or 8 times daily, with a single dose of 5 µL per eye, for a duration of 3 weeks.

In addition to measuring electroretinogram (ERG) signals (a- and b-waves) of the experimental animals on day 0, day 14, and day 35, eyeball samples were collected after the animals were sacrificed at the experimental endpoint. Finally, based on the results of immunofluorescence (IF) staining, the expression of clusterin, which is a biomarker of drusen, was evaluated and quantitatively analyzed.

The results are shown in FIGs. 2B and 2C (administration of 5 µL per eye of the previously prepared eye drops containing sodium phenylbutyrate (SPB) at a frequency of 4 times daily) and FIGs. 3A to 3C (administration of 5 µL per eye of the previously prepared eye drops containing sodium phenylbutyrate (SPB) at a frequency of 8 times daily).

FIG. 2B shows the immunofluorescence staining results of mouse eye samples in the hydroquinone (HQ)-induced animal disease model experiment, in which arrows indicate regions of clusterin expression. FIG. 2C shows the quantified expression levels of clusterin in mouse eye samples in the hydroquinone (HQ)-induced animal disease model experiment.

According to FIGs. 2B and 2C, compared with the untreated group, the prepared eye drops containing sodium phenylbutyrate (SPB) at a concentration of greater than 30 mg/mL effectively reduced clusterin expression in the sub-retinal pigment epithelium (sub-RPE) under hydroquinone (HQ)-induced retinal degeneration, and the inhibitory effect exhibited a dose-dependent trend.

FIGs. 3A to 3C respectively show the electroretinogram (ERG) signals (a- and b-waves) of the experimental animals on day 0, day 14, and day 35.

According to the electroretinograms shown in FIGs. 3A to 3C, compared with the untreated group, the prepared eye drops containing sodium phenylbutyrate (SPB) at a concentration of greater than or equal to 60 mg/mL effectively restored retinal functional impairment caused by hydroquinone (HQ) under hydroquinone (HQ)-induced retinal degeneration, and the therapeutic effect exhibited a dose-dependent trend.

While the invention has been described by way of example and in terms of the preferred embodiments, it should be understood that the invention is not limited to the disclosed embodiments. On the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. An eye drop platform for delivering an active ingredient to anterior chamber tissue and/or posterior chamber tissue of an eye, comprising:
a delivery composition, which comprises:
cyclodextrin and/or a derivative thereof; and
caffeine; and
a pharmaceutically acceptable solvent for dissolving the delivery composition to form a solution, wherein the solution serves as the eye drop platform for delivering an active ingredient to anterior chamber tissue and/or posterior chamber tissue of an eye,
wherein:
a concentration of the cyclodextrin and/or the derivative thereof in the solution ranges from 5-500 mg/mL; and
a concentration of the caffeine in the solution ranges from 0.5-50 mg/mL; and
wherein the active ingredient is a hydrophilic compound having a water solubility greater than 1 mg/mL.

2. The eye drop platform for delivering the active ingredient to anterior chamber tissue and/or posterior chamber tissue of the eye of claim 1, wherein
the cyclodextrin comprises at least one of the following:
α-cyclodextrin;
β-cyclodextrin;
γ-cyclodextrin; and
δ-cyclodextrin, and
the derivative of the cyclodextrin comprises at least one of the following:
hydroxypropyl-modified cyclodextrin;
succinyl-modified cyclodextrin; and
methyl-modified cyclodextrin,
wherein the hydroxypropyl-modified cyclodextrin comprises:
(2-hydroxypropyl)-β-cyclodextrin; and/or
(2-hydroxypropyl)-γ-cyclodextrin.

3. The eye drop platform for delivering the active ingredient to anterior chamber tissue and/or posterior chamber tissue of the eye of claim 1 or 2, wherein the delivery composition further comprises a water-soluble polymer, and a concentration of the water-soluble polymer in the solution ranges from 0.5-20 mg/mL,
wherein the water-soluble polymer comprises at least one of the following:
hydroxypropyl methyl cellulose;
hydroxypropyl cellulose;
carboxymethyl cellulose;
polyvinylpyrrolidone;
polyvinyl alcohol; and
poly(ethylene glycol)-poly(propylene glycol)-poly(ethylene glycol) triblock copolymer.

4. An eye drop for improving exposure of an active ingredient in anterior chamber tissue and/or posterior chamber tissue of an eye, comprising:
a delivery composition which comprises:
cyclodextrin and/or a derivative thereof; and
caffeine; and
a pharmaceutically acceptable solvent for dissolving the delivery composition to form a solution, wherein the solution serves as the eye drop for improving exposure of an active ingredient in anterior chamber tissue and/or posterior chamber tissue of an eye,
wherein:
a concentration of the cyclodextrin and/or the derivative thereof in the eye drop ranges from 5-500 mg/mL; and
a concentration of the caffeine in the eye drop ranges from 0.5-50 mg/mL; and
wherein the active ingredient is a hydrophilic compound having a water solubility greater than 1 mg/mL.

5. The eye drop for improving exposure of the active ingredient in anterior chamber tissue and/or posterior chamber tissue of the eye of claim 4, wherein
the cyclodextrin comprises at least one of the following:
α-cyclodextrin;
β-cyclodextrin;
γ-cyclodextrin; and
δ-cyclodextrin, and
the derivative of the cyclodextrin comprises at least one of the following:
hydroxypropyl-modified cyclodextrin;
succinyl-modified cyclodextrin; and
methyl-modified cyclodextrin,
wherein the hydroxypropyl-modified cyclodextrin comprises:
(2-hydroxypropyl)-β-cyclodextrin; and/or
(2-hydroxypropyl)-γ-cyclodextrin.

6. The eye drop for improving exposure of the active ingredient in anterior chamber tissue and/or posterior chamber tissue of the eye of claim 4 or 5, wherein the delivery composition further comprises a water-soluble polymer, and a concentration of the water-soluble polymer in the solution ranges from 0.5-20 mg/mL,
wherein the water-soluble polymer comprises at least one of the following:
hydroxypropyl methyl cellulose;
hydroxypropyl cellulose;
carboxymethyl cellulose;
polyvinylpyrrolidone;
polyvinyl alcohol; and
poly(ethylene glycol)-poly(propylene glycol)-poly(ethylene glycol) triblock copolymer.

7. A composite formulation, comprising:
an active ingredient, wherein the active ingredient is a hydrophilic compound having a water solubility greater than 1 mg/mL;
a delivery composition, comprising:
cyclodextrin and/or a derivative thereof; and
caffeine; and
a pharmaceutically acceptable solvent for dissolving the active ingredient and the delivery composition to form a solution, wherein the solution serves as the composite formulation,
wherein:
a concentration of the active ingredient in the solution ranges from 0.05-500 mg/mL;
a concentration of the cyclodextrin and/or the derivative thereof in the solution ranges from 5-500 mg/mL;
a concentration of the caffeine in the solution ranges from 0.5-50 mg/mL; and
wherein the composite formulation is an eye drop.

8. The composite formulation of claim 7, wherein the active ingredient comprises at least one of the following:
a drug having a neuroprotective function;
a complement inhibitor;
an AMP-activated protein kinase (AMPK) activator; and
a Rho-associated coiled-coil kinase inhibitor.

9. The composite formulation of claim 8, wherein
the drug having the neuroprotective function comprises at least one of the following:
sodium phenylbutyrate (SPB);
dexmedetomidine HCl; and
tauro-ursodesoxy cholic acid (TUDCA),
the complement inhibitor comprises iptacopan (LNP-023) and/or nafamostat mesylate (FUT-175),
the AMP-activated protein kinase activator comprises a biguanide drug, and the biguanide drug comprises metformin HCl, and
the Rho-associated coiled-coil kinase inhibitor comprises (S)-N-(4-(1H-pyrazol-4-yl)phenyl)-3-amino-2-phenylpropanamide dihydrochloride (ITRI-E-(S)4046).

10. The composite formulation of any one of claims 7-9, wherein
the cyclodextrin comprises at least one of the following:
α-cyclodextrin;
β-cyclodextrin;
γ-cyclodextrin; and
δ-cyclodextrin, and
the derivative of the cyclodextrin comprises at least one of the following:
hydroxypropyl-modified cyclodextrin;
succinyl-modified cyclodextrin; and
methyl-modified cyclodextrin,
wherein the hydroxypropyl-modified cyclodextrin comprises:
(2-hydroxypropyl)-β-cyclodextrin; and/or
(2-hydroxypropyl)-γ-cyclodextrin.

11. The composite formulation of any one of claims 7-10, wherein the delivery composition further comprises a water-soluble polymer, and a concentration of the water-soluble polymer in the solution ranges from 0.5-20 mg/mL,
wherein the water-soluble polymer comprises at least one of the following:
hydroxypropyl methyl cellulose;
hydroxypropyl cellulose;
carboxymethyl cellulose;
polyvinylpyrrolidone;
polyvinyl alcohol; and
poly(ethylene glycol)-poly(propylene glycol)-poly(ethylene glycol) triblock copolymer.

12. An eye drop for treating and/or preventing an anterior chamber disease and/or a posterior chamber disease of an eye, comprising:
the composite formulation of any one of claims 7-11.

13. The eye drop for treating and/or preventing the anterior chamber disease and/or the posterior chamber disease of the eye of claim 12, wherein the active ingredient comprises at least one of the following:
a drug having a neuroprotective function;
a complement inhibitor;
an AMP-activated protein kinase (AMPK) activator; and
a Rho-associated coiled-coil kinase inhibitor.

14. The eye drop for treating and/or preventing the anterior chamber disease and/or the posterior chamber disease of the eye of claim 13, wherein
the drug having the neuroprotective function comprises at least one of the following:
sodium phenylbutyrate (SPB);
dexmedetomidine HCl; and
tauro-ursodesoxy cholic acid (TUDCA),
the complement inhibitor comprises iptacopan (LNP-023) and/or nafamostat mesylate (FUT-175),
the AMP-activated protein kinase activator comprises a biguanide drug, and the biguanide drug comprises metformin HCl, and
the Rho-associated coiled-coil kinase inhibitor comprises (S)-N-(4-(1H-pyrazol-4-yl)phenyl)-3-amino-2-phenylpropanamide dihydrochloride (ITRI-E-(S)4046).

15. The eye drop for treating and/or preventing the anterior chamber disease and/or the posterior chamber disease of the eye of any one of claims 12-14, wherein
the cyclodextrin comprises at least one selected from the group consisting of:
α-cyclodextrin;
β-cyclodextrin;
γ-cyclodextrin; and
δ-cyclodextrin, and
the derivative of the cyclodextrin comprises at least one of the following:
hydroxypropyl-modified cyclodextrin;
succinyl-modified cyclodextrin; and
methyl-modified cyclodextrin,
wherein the hydroxypropyl-modified cyclodextrin comprises:
(2-hydroxypropyl)-β-cyclodextrin; and/or
(2-hydroxypropyl)-γ-cyclodextrin.

16. The eye drop for treating and/or preventing the anterior chamber disease and/or the posterior chamber disease of the eye of any one of claims 12-15, wherein the delivery composition further comprises a water-soluble polymer, and a concentration of the water-soluble polymer in the solution ranges from 0.5-20 mg/mL,
wherein the water-soluble polymer comprises at least one of the following:
hydroxypropyl methyl cellulose;
hydroxypropyl cellulose;
carboxymethyl cellulose;
polyvinylpyrrolidone;
polyvinyl alcohol; and
poly(ethylene glycol)-poly(propylene glycol)-poly(ethylene glycol) triblock copolymer.

17. The eye drop for treating and/or preventing the anterior chamber disease and/or the posterior chamber disease of the eye of any one of claims 12-16, wherein
the anterior chamber disease comprises at least one of the following:
glaucoma;
anterior uveitis;
cataracts; and
dry eye,
the posterior chamber disease comprises at least one of the following:
age-related macular degeneration (AMD);
diabetic macular edema; and
diabetic retinopathy.

18. An eye drop for treating and/or preventing age-related macular degeneration, comprising:
a composite formulation, which comprise:
sodium phenylbutyrate;
a delivery composition, which comprises:
(2-hydroxypropyl)-β-cyclodextrin and/or (2-hydroxypropyl)-γ-cyclodextrin; and
caffeine; and
a pharmaceutically acceptable solvent for dissolving the sodium phenylbutyrate and the delivery composition to form a solution, wherein the solution serves as the composite formulation,
wherein:
a concentration of the sodium phenylbutyrate in the solution ranges from 0.05-150 mg/mL;
a concentration of the (2-hydroxypropyl)-β-cyclodextrin and/or the (2-hydroxypropyl)-γ-cyclodextrin in the solution ranges from 5-500 mg/mL; and
a concentration of the caffeine in the solution ranges from 0.5-50 mg/mL.

19. The eye drop for treating and/or preventing age-related macular degeneration of claim 18, wherein the delivery composition further comprises hydroxypropyl methyl cellulose, and a concentration of the hydroxypropyl methyl cellulose in the solution ranges from 0.5-20 mg/mL.
